(19) Europäisches Patentamt / European Patent Office / Office européen des brevets

(11) EP 4 183 420 A1

(12) EUROPEAN PATENT APPLICATION
published in accordance with Art. 153(4) EPC

(43) Date of publication:
**24.05.2023 Bulletin 2023/21**

(21) Application number: **21846150.7**

(22) Date of filing: **19.07.2021**

(51) International Patent Classification (IPC):
***A61K 47/68*** (2017.01) ***A61K 31/4745*** (2006.01)
***A61K 39/395*** (2006.01) ***A61P 35/00*** (2006.01)
***A61P 35/02*** (2006.01) ***A61P 43/00*** (2006.01)
***C07K 16/30*** (2006.01) ***C12N 15/13*** (2006.01)

(52) Cooperative Patent Classification (CPC):
**A61K 31/4745; A61K 39/395; A61K 47/68; A61P 35/00; A61P 35/02; A61P 43/00; C07K 16/00; C07K 16/30**

(86) International application number:
**PCT/JP2021/026932**

(87) International publication number:
**WO 2022/019259 (27.01.2022 Gazette 2022/04)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **20.07.2020 JP 2020123564**

(71) Applicant: **Daiichi Sankyo Company, Limited**
**Tokyo 103-8426 (JP)**

(72) Inventor: **KAMAI Yasuki**
**Tokyo 103-8426 (JP)**

(74) Representative: **Marks & Clerk LLP**
**15 Fetter Lane**
**London EC4A 1BW (GB)**

Remarks:
The complete document including Reference Table(s) and the Sequence Listing(s) can be downloaded from the EPO website

# (54) COMBINATION OF (ANTI-HER2 ANTIBODY)-DRUG CONJUGATE AND HER DIMERIZATION INHIBITOR

(57) A pharmaceutical composition, wherein an anti-HER2 antibody-drug conjugate in which a drug-linker represented by the following formula (wherein A represents a connecting position to an anti-HER2 antibody) is conjugated to the anti-HER2 antibody via a thioether bond, and a HER dimerization inhibitor are administered in combination, and/or a method of treatment, wherein the anti-HER2 antibody-drug conjugate and a HER dimerization inhibitor are administrated in combination to a subject.

[Figure 13]

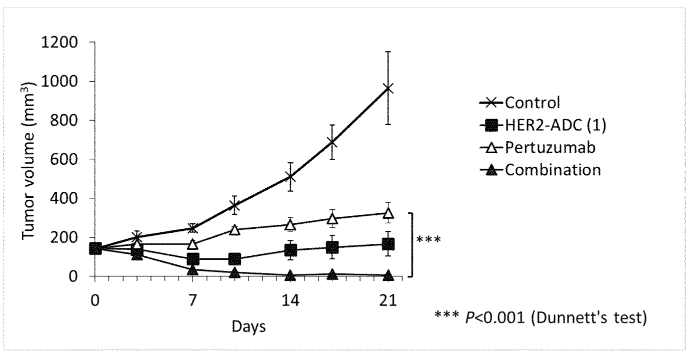




EP 4 183 420 A1

## Description

### Technical Field

The present invention relates to a pharmaceutical composition, wherein a specific anti-HER2 antibody-drug conjugate and a HER dimerization inhibitor are administered in combination, and/or a method of treatment, comprising administering a specific anti-HER2 antibody-drug conjugate and a HER dimerization inhibitor in combination to a subject.

### Background Art

Human epidermal growth factor receptor 2 (HER2) is a transmembrane receptor belonging to the epidermal growth factor receptor subfamily of receptor tyrosine kinases (Non-Patent References 1 to 6). HER2 is overexpressed in various types of cancer including breast cancer and gastric cancer (Non-Patent References 7 to 12), and has been reported to be a negative prognostic factor for breast cancer (Non-Patent References 13, 14).

Known anti-HER2 antibodies include trastuzumab (Patent Reference 1) and pertuzumab (Patent Reference 2).

Trastuzumab is known to bind to subdomain IV of the extracellular domain of HER2 protein.

On the other hand, pertuzumab is known to bind to subdomain II of the extracellular domain of HER2 protein. This is expected to inhibit the dimerization of HER2 protein and HER3 protein to inhibit phosphorylation of HER2 by ligand stimulation and activation of the kinases PI3K-Akt and MAPK, which are present in the downstream, thereby suppressing growth of cells. From this expectation, pertuzumab is known as a HER dimerization inhibitor.

Trastuzumab is known to exert a further superior antitumor effect when used in combination with pertuzumab (Non-Patent References 15, 16).

An antibody-drug conjugate (ADC) having a drug with cytotoxicity conjugated to an antibody capable of binding to an antigen expressed on the surface of cancer cells and cellular internalization, can deliver the drug selectively to cancer cells and can thus be expected to cause accumulation of the drug within cancer cells and to kill the cancer cells (Non-Patent References 17 to 21).

As one such antibody-drug conjugate, an antibody-drug conjugate comprising an anti-HER2 antibody and a derivative of exatecan, which is a topoisomerase I inhibitor, as its components is known (Patent References 3 to 6, Non-Patent References 22 to 25).

### Citation List

#### Patent Literature

Patent Reference 1: U.S. Patent No. 5821337
Patent Reference 2: International Publication No. WO 01/00245
Patent Reference 3: International Publication No. WO 2015/115091
Patent Reference 4: International Publication No. WO 2015/155976
Patent Reference 5: International Publication No. WO 2018/066626
Patent Reference 6: International Publication No. WO 2019/230645

#### Non-Patent Literature

Non-Patent Reference 1: Coussens L, et al., Science. 1985; 230(4730): 1132-1139.
Non-Patent Reference 2: Graus-Porta G, et al., EMBO J. 1997;16:1647-1655.
Non-Patent Reference 3: Karnagaran D, et al., EMBO J. 1996;15:254-264.
Non-Patent Reference 4: Sliwkowski MX, et al., J Biom Chem. 1994; 269: 14661-14665.
Non-Patent Reference 5: Di Fore PP, et al., Science. 1987; 237: 178-182.
Non-Patent Reference 6: Hudziak RM, et al., Proc Natl Acad Sci USA. 1987; 84: 7159-7163.
Non-Patent Reference 7: Hardwick R, et al., Eur. J Surg Oncol. 1997 (23):30-35.
Non-Patent Reference 8: Korkaya H, et al., Oncogene. 2008;27(47):6120-6130.
Non-Patent Reference 9: Yano T, et al., Oncol Rep. 2006; 15(1): 65-71.
Non-Patent Reference 10: Slamon DJ, et al., Science. 1987; 235: 177-182.
Non-Patent Reference 11: Gravalos C, et al., Ann Oncol 19: 1523-1529, 2008.

Non-Patent Reference 12: Fukushige S et al., Mol Cell Biol 6: 955-958, 1986.
Non-Patent Reference 13: Slamon DJ, et al., Science. 1989; 244: 707-712.
Non-Patent Reference 14: Kaptain S, et al., Diagn Mol Pathol 10: 139-152, 2001.
Non-Patent Reference 15: Richard S, et al., An Acad Bras Cienc. 2016;88 Suppl 1:565-77.
Non-Patent Reference 16: Nami B, et al., Cancers (Basel). 2019 Mar 16;11(3):375.
Non-Patent Reference 17: Ducry, L., et al., Bioconjugate Chem. (2010) 21, 5-13.
Non-Patent Reference 18: Alley, S. C., et al., Current Opinion in Chemical Biology (2010) 14, 529-537.
Non-Patent Reference 19: Damle N. K. Expert Opin. Biol. Ther. (2004) 4, 1445-1452.
Non-Patent Reference 20: Senter P. D., et al., Nature Biotechnology (2012) 30, 631-637.
Non-Patent Reference 21: Howard A. et al., J Clin Oncol 29: 398-405.
Non-Patent Reference 22: Ogitani Y. et al., Clinical Cancer Research (2016) 22 (20), 5097-5108.
Non-Patent Reference 23: Ogitani Y. et al., Cancer Science (2016) 107, 1039-1046.
Non-Patent Reference 24: Doi T, et al., Lancet Oncol. (2017) 18, 1512-22.
Non-Patent Reference 25: Takegawa N, et al., Int. J. Cancer (2017) 141, 1682-1689.

## Summary of Invention

### Technical Problem

Expectation of an antitumor effect like combination therapy with trastuzumab and pertuzumab is driving ongoing research on use of an anti-HER2 antibody-drug conjugate and a HER dimerization inhibitor in combination (see Oncol Rep. 2013 Sep; 30(3): 1087-93, and Clin Cancer Res. 2014 Jan 15; 20(2): 456-68, etc.).

However, it has been reported that, on the contrary, no expected antitumor effect was found for use of trastuzumab emtansine, which is an anti-HER2 antibody-drug conjugate, and pertuzumab in combination (see Oncotarget. 2018 Aug 7; 9(61): 31915-31919, etc.).

Thus, combination therapy with an anti-HER2 antibody-drug conjugate and a HER dimerization inhibitor has not been established yet.

An object of the present invention is to provide a pharmaceutical composition, wherein a specific anti-HER2 antibody-drug conjugate and a HER dimerization inhibitor are administered in combination, and/or a method of treatment, wherein the anti-HER2 antibody-drug conjugate and a HER dimerization inhibitor are administered in combination to a subject.

### Solution to Problem

As a result of diligent studies in order to solve the above problems, the present inventors have found that combined administration of a specific anti-HER2 antibody-drug conjugate and a HER dimerization inhibitor exhibits a superior combined effect, and thereby completed the present invention.

Thus, the present invention provides the following [1] to [100].

[1] A pharmaceutical composition, wherein an anti-HER2 antibody-drug conjugate and a HER dimerization inhibitor are administered in combination, and the anti-HER2 antibody-drug conjugate is an anti-HER2 antibody-drug conjugate in which a drug-linker represented by the following formula:

[Formula 1]

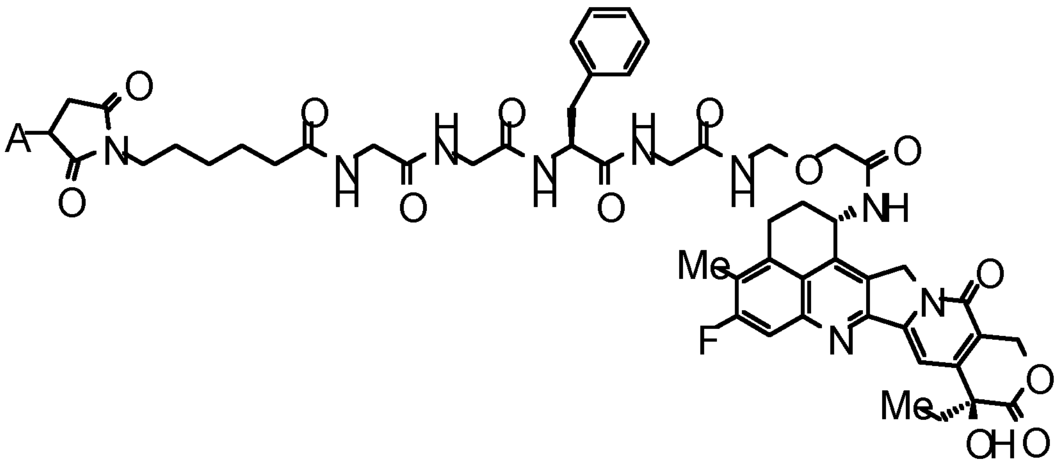

wherein A represents a connecting position to an anti-HER2 antibody, is conjugated to the anti-HER2 antibody via a thioether bond.

[2] The pharmaceutical composition according to [1], wherein the anti-HER2 antibody is an antibody comprising a heavy chain comprising CDRH1 consisting of an amino acid sequence represented by of SEQ ID NO: 3, CDRH2 consisting of an amino acid sequence represented by SEQ ID NO: 4, and CDRH3 consisting of an amino acid sequence represented by SEQ ID NO: 5, and a light chain comprising CDRL1 consisting of an amino acid sequence represented by SEQ ID NO: 6, CDRL2 consisting of an amino acid sequence represented by amino acid residues 1 to 3 of SEQ ID NO: 7, and CDRL3 consisting of an amino acid sequence represented by SEQ ID NO: 8.
[3] The pharmaceutical composition according to [1], wherein the anti-HER2 antibody is an antibody comprising a heavy chain comprising a heavy chain variable region consisting of an amino acid sequence represented by SEQ ID NO: 9 and a light chain comprising a light chain variable region consisting of an amino acid sequence represented by SEQ ID NO: 10.
[4] The pharmaceutical composition according to [1], wherein the anti-HER2 antibody is an antibody comprising a heavy chain consisting of an amino acid sequence represented by SEQ ID NO: 1 and a light chain consisting of an amino acid sequence represented by SEQ ID NO: 2.
[5] The pharmaceutical composition according to [1], wherein the anti-HER2 antibody is an antibody comprising a heavy chain consisting of an amino acid sequence represented by SEQ ID NO: 11 and a light chain consisting of an amino acid sequence represented by SEQ ID NO: 2.
[6] The pharmaceutical composition according to any one of [1] to [5], wherein the anti-HER2 antibody-drug conjugate is an anti-HER2 antibody-drug conjugate represented by the following formula:

[Formula 2]

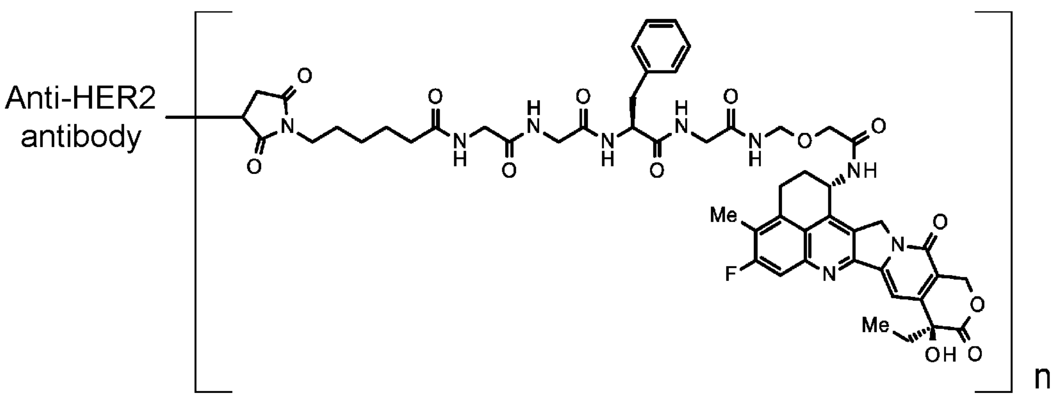

wherein a drug-linker is conjugated to the anti-HER2 antibody via a thioether bond; and n represents the average

number of units of the drug-linker conjugated per antibody molecule, wherein n is in the range of from 7 to 8.

[7] The pharmaceutical composition according to any one of [1] to [6], wherein the anti-HER2 antibody-drug conjugate is trastuzumab deruxtecan.

[8] The pharmaceutical composition according to any one of [1] to [7], wherein the HER dimerization inhibitor is an antibody capable of binding to subdomain II of an extracellular domain of HER2 protein.

[9] The pharmaceutical composition according to any one of [1] to [7], wherein the HER dimerization inhibitor is pertuzumab.

[10] The pharmaceutical composition according to any one of [1] to [9], wherein the anti-HER2 antibody-drug conjugate and the HER dimerization inhibitor are separately contained as active components in different formulations, and are administered simultaneously or at different times.

[11] The pharmaceutical composition according to any one of [1] to [9], wherein the anti-HER2 antibody-drug conjugate and the HER dimerization inhibitor are contained as active components in a single formulation and administered.

[12] The pharmaceutical composition according to any one of [1] to [11] wherein the pharmaceutical composition is for use in treating cancer.

[13] The pharmaceutical composition according to [12], wherein the cancer is at least one selected from the group consisting of breast cancer, gastric cancer, colorectal cancer, lung cancer, esophageal cancer, head-and-neck cancer, gastroesophageal junction adenocarcinoma, biliary tract cancer, Paget's disease, pancreatic cancer, ovarian cancer, uterine carcinosarcoma, bladder cancer, prostate cancer, urothelial cancer, gastrointestinal stromal tumor, uterine cervix cancer, squamous cell carcinoma, peritoneal cancer, liver cancer, hepatocellular cancer, endometrial cancer, kidney cancer, vulval cancer, thyroid cancer, penis cancer, leukemia, malignant lymphoma, plasmacytoma, myeloma, glioblastoma multiforme, sarcoma, osteosarcoma, and melanoma.

[14] The pharmaceutical composition according to [12], wherein the cancer is at least one selected from the group consisting of breast cancer, gastric cancer, colorectal cancer, lung cancer, esophageal cancer, head-and-neck cancer, gastroesophageal junction adenocarcinoma, biliary tract cancer, Paget's disease, pancreatic cancer, ovarian cancer, uterine carcinosarcoma, bladder cancer, and prostate cancer.

[15] The pharmaceutical composition according to [12], wherein the cancer is breast cancer.

[16] The pharmaceutical composition according to any one of [12] to [15], wherein the cancer is HER2-overexpressing cancer.

[17] The pharmaceutical composition according to [16], wherein the HER2-overexpressing cancer is cancer given a score of 3+ for the expression of HER2 in an immunohistochemical method.

[18] The pharmaceutical composition according to [16], wherein the HER2-overexpressing cancer is cancer given a score of 2+ for the expression of HER2 in an immunohistochemical method and determined as positive for the expression of HER2 in an *in situ* hybridization method.

[19] The pharmaceutical composition according to any one of [12] to [15], wherein the cancer is HER2 low-expressing cancer.

[20] The pharmaceutical composition according to [19], wherein the HER2 low-expressing cancer is cancer given a score of 2+ for the expression of HER2 in an immunohistochemical method and determined as negative for the expression of HER2 in an *in situ* hybridization method.

[21] The pharmaceutical composition according to [19], wherein the HER2 low-expressing cancer is cancer given a score of 1+ for the expression of HER2 in an immunohistochemical method.

[22] The pharmaceutical composition according to [19], wherein the HER2 low-expressing cancer is cancer given a score of >0 and <1+ for the expression of HER2 in an immunohistochemical method.

[23] The pharmaceutical composition according to any one of [12] to [22], wherein the pharmaceutical composition exerts a significantly superior antitumor effect than single administration of the anti-HER2 antibody-drug conjugate.

[24] The pharmaceutical composition according to any one of [12] to [23], wherein the pharmaceutical composition is for use in treating cancer untreatable with single administration of the anti-HER2 antibody-drug conjugate.

[25] The pharmaceutical composition according to any one of [12] to [24], wherein the anti-HER2 antibody-drug conjugate and the HER dimerization inhibitor exert a synergistic antitumor effect.

[26] A method of treatment, comprising administering an anti-HER2 antibody-drug conjugate and a HER dimerization inhibitor in combination to a subject in need of the treatment, wherein the anti-HER2 antibody-drug conjugate is an anti-HER2 antibody-drug conjugate in which a drug-linker represented by the following formula:

[Formula 3]

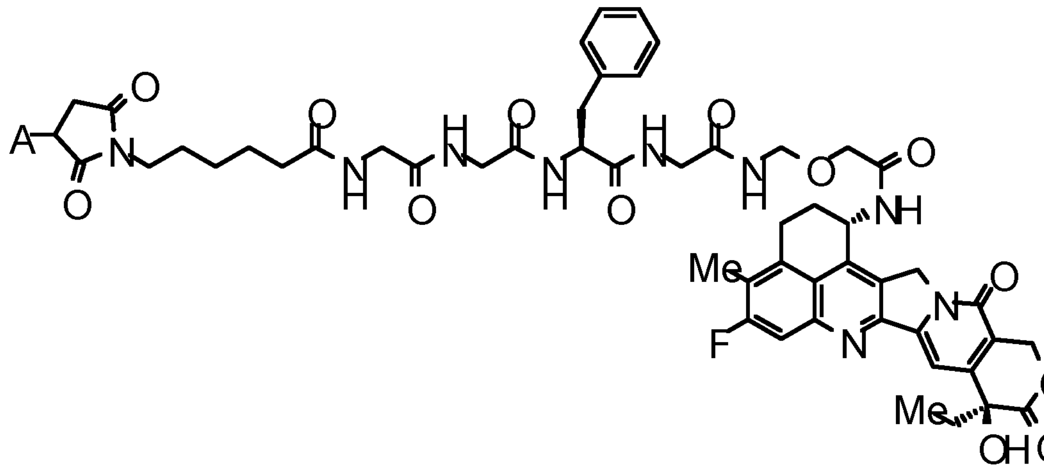

wherein A represents a connecting position to an anti-HER2 antibody,
is conjugated to the anti-HER2 antibody via a thioether bond.

[27] The method of treatment according to [26], wherein the anti-HER2 antibody is an antibody comprising a heavy chain comprising CDRH1 consisting of an amino acid sequence represented by SEQ ID NO: 3, CDRH2 consisting of an amino acid sequence represented by SEQ ID NO: 4, and CDRH3 consisting of an amino acid sequence represented by SEQ ID NO: 5, and a light chain comprising CDRL1 consisting of an amino acid sequence represented by SEQ ID NO: 6, CDRL2 consisting of an amino acid sequence represented by amino acid residues 1 to 3 of SEQ ID NO: 7, and CDRL3 consisting of an amino acid sequence represented by of SEQ ID NO: 8.
[28] The method of treatment according to [26], wherein the anti-HER2 antibody is an antibody comprising a heavy chain comprising a heavy chain variable region consisting of an amino acid sequence represented by SEQ ID NO: 9 and a light chain comprising a light chain variable region consisting of an amino acid sequence represented by SEQ ID NO: 10.
[29] The method of treatment according to [26], wherein the anti-HER2 antibody is an antibody comprising a heavy chain consisting of an amino acid sequence represented by SEQ ID NO: 1 and a light chain consisting of an amino acid sequence represented by SEQ ID NO: 2.
[30] The method of treatment according to [26], wherein the anti-HER2 antibody is an antibody comprising a heavy chain consisting of an amino acid sequence represented by SEQ ID NO: 11 and a light chain consisting of an amino acid sequence represented by SEQ ID NO: 2.
[31] The method of treatment according to any one of [26] to [30], wherein the anti-HER2 antibody-drug conjugate is an anti-HER2 antibody-drug conjugate represented by the following formula:

[Formula 4]

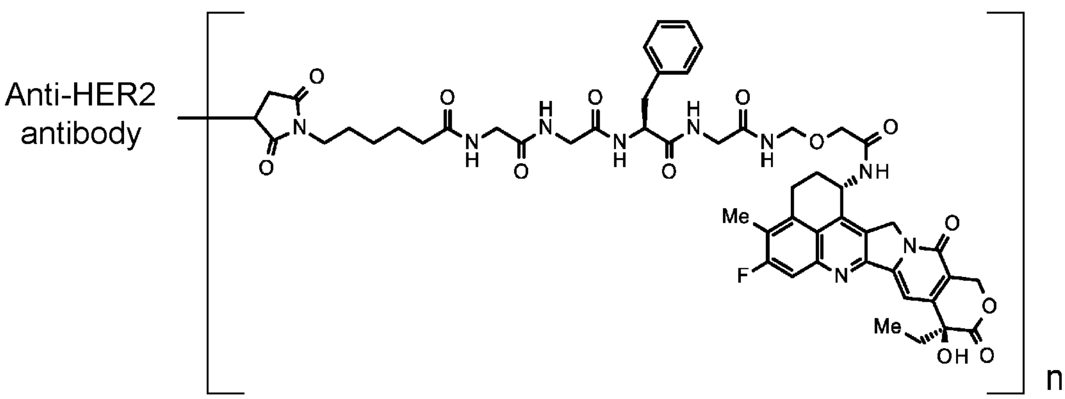

wherein a drug-linker is conjugated to the anti-HER2 antibody via a thioether bond; and n represents the average number of units of the drug-linker conjugated per antibody molecule, wherein n is in the range of from 7 to 8.

[32] The method of treatment according to any one of [26] to [31], wherein the anti-HER2 antibody-drug conjugate is trastuzumab deruxtecan.

[33] The method of treatment according to any one of [26] to [32], wherein the HER dimerization inhibitor is an antibody capable of binding to subdomain II of an extracellular domain of HER2 protein.

[34] The method of treatment according to any one of [26] to [32], wherein the HER dimerization inhibitor is pertuzumab.

[35] The method of treatment according to any one of [26] to [34], wherein the anti-HER2 antibody-drug conjugate and the HER dimerization inhibitor are separately contained as active components in different formulations, and are administered simultaneously or at different times.

[36] The method of treatment according to any one of [26] to [34], wherein the anti-HER2 antibody-drug conjugate and the HER dimerization inhibitor are contained as active components in a single formulation and administered.

[37] The method of treatment according to any one of [26] to [36], wherein the method is for use in treating cancer.

[38] The method of treatment according to [37], wherein the cancer is at least one selected from the group consisting of breast cancer, gastric cancer, colorectal cancer, lung cancer, esophageal cancer, head-and-neck cancer, gastroesophageal junction adenocarcinoma, biliary tract cancer, Paget's disease, pancreatic cancer, ovarian cancer, uterine carcinosarcoma, bladder cancer, prostate cancer, urothelial cancer, gastrointestinal stromal tumor, uterine cervix cancer, squamous cell carcinoma, peritoneal cancer, liver cancer, hepatocellular cancer, endometrial cancer, kidney cancer, vulval cancer, thyroid cancer, penis cancer, leukemia, malignant lymphoma, plasmacytoma, myeloma, glioblastoma multiforme, sarcoma, osteosarcoma, and melanoma.

[39] The method of treatment according to [37], wherein the cancer is at least one selected from the group consisting of breast cancer, gastric cancer, colorectal cancer, lung cancer, esophageal cancer, head-and-neck cancer, gastroesophageal junction adenocarcinoma, biliary tract cancer, Paget's disease, pancreatic cancer, ovarian cancer, uterine carcinosarcoma, bladder cancer, and prostate cancer.

[40] The method of treatment according to [37], wherein the cancer is breast cancer.

[41] The method of treatment according to any one of [37] to [40], wherein the cancer is HER2-overexpressing cancer.

[42] The method of treatment according to [41], wherein the HER2-overexpressing cancer is cancer given a score of 3+ for the expression of HER2 in an immunohistochemical method.

[43] The method of treatment according to [41], wherein the HER2-overexpressing cancer is cancer given a score of 2+ for the expression of HER2 in an immunohistochemical method and determined as positive for the expression of HER2 in an *in situ* hybridization method.

[44] The method of treatment according to any one of [37] to [40], wherein the cancer is HER2 low-expressing cancer.

[45] The method of treatment according to [44], wherein the HER2 low-expressing cancer is cancer given a score of 2+ for the expression of HER2 in an immunohistochemical method and determined as negative for the expression of HER2 in an *in situ* hybridization method.

[46] The method of treatment according to [44], wherein the HER2 low-expressing cancer is cancer given a score of 1+ for the expression of HER2 in an immunohistochemical method.

[47] The method of treatment according to [44], wherein the HER2 low-expressing cancer is cancer given a score of >0 and <1+ for the expression of HER2 in an immunohistochemical method.

[48] The method of treatment according to any one of [37] to [47], which exerts a significantly superior antitumor effect than single administration of the anti-HER2 antibody-drug conjugate.

[49] The method of treatment according to any one of [37] to [48], wherein the method is for use in treating cancer untreatable with single administration of the anti-HER2 antibody-drug conjugate.

[50] The method of treatment according to any one of [37] to [49], wherein the anti-HER2 antibody-drug conjugate and the HER dimerization inhibitor exert a synergistic antitumor effect.

[51] An anti-HER2 antibody-drug conjugate for use in treating a disease through being administered in combination with a HER dimerization inhibitor, wherein a drug-linker represented by the following formula:

[Formula 5]

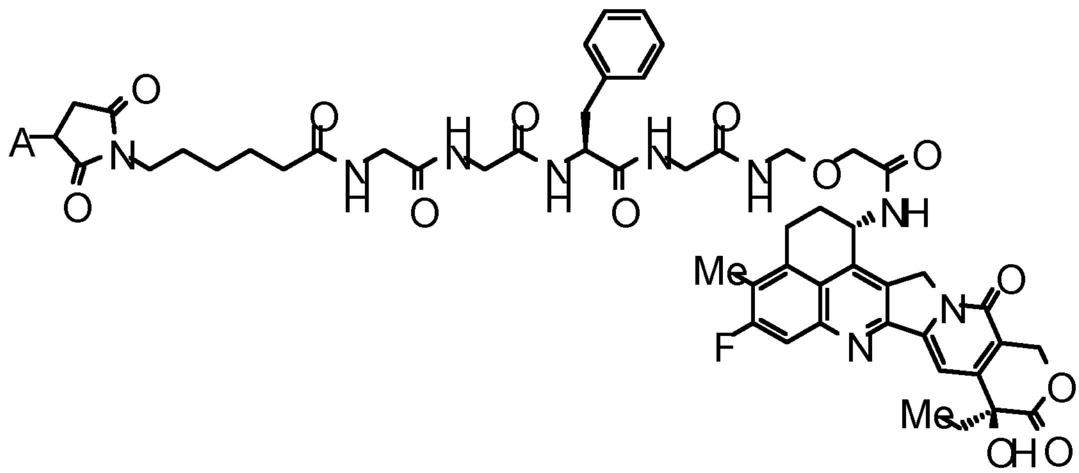

wherein A represents a connecting position to an anti-HER2 antibody,
is conjugated to the anti-HER2 antibody via a thioether bond in the anti-HER2 antibody-drug conjugate.

[52] The anti-HER2 antibody-drug conjugate according of [51], wherein the anti-HER2 antibody is an antibody comprising a heavy chain comprising CDRH1 consisting of an amino acid sequence represented by SEQ ID NO: 3, CDRH2 consisting of an amino acid sequence represented by SEQ ID NO: 4, and CDRH3 consisting of an amino acid sequence represented by SEQ ID NO: 5, and a light chain comprising CDRL1 consisting of an amino acid sequence represented by SEQ ID NO: 6, CDRL2 consisting of an amino acid sequence consisting of amino acid residues 1 to 3 of SEQ ID NO: 7, and CDRL3 consisting of an amino acid sequence represented by SEQ ID NO: 8.
[53] The anti-HER2 antibody-drug conjugate according of [51], wherein the anti-HER2 antibody is an antibody comprising a heavy chain comprising a heavy chain variable region consisting of an amino acid sequence represented by SEQ ID NO: 9, and a light chain comprising a light chain variable region consisting of an amino acid sequence represented by SEQ ID NO: 10.
[54] The anti-HER2 antibody-drug conjugate according of [51], wherein the anti-HER2 antibody is an antibody comprising a heavy chain consisting of an amino acid sequence represented by SEQ ID NO: 1 and a light chain consisting of an amino acid sequence represented by SEQ ID NO: 2.
[55] The anti-HER2 antibody-drug conjugate according of [51], wherein the anti-HER2 antibody is an antibody comprising a heavy chain consisting of an amino acid sequence represented by SEQ ID NO: 11 and a light chain consisting of an amino acid sequence represented by SEQ ID NO: 2.
[56] The anti-HER2 antibody-drug conjugate according to any one of [51] to [55], wherein the anti-HER2 antibody-drug conjugate is an anti-HER2 antibody-drug conjugate represented by the following formula:

[Formula 6]

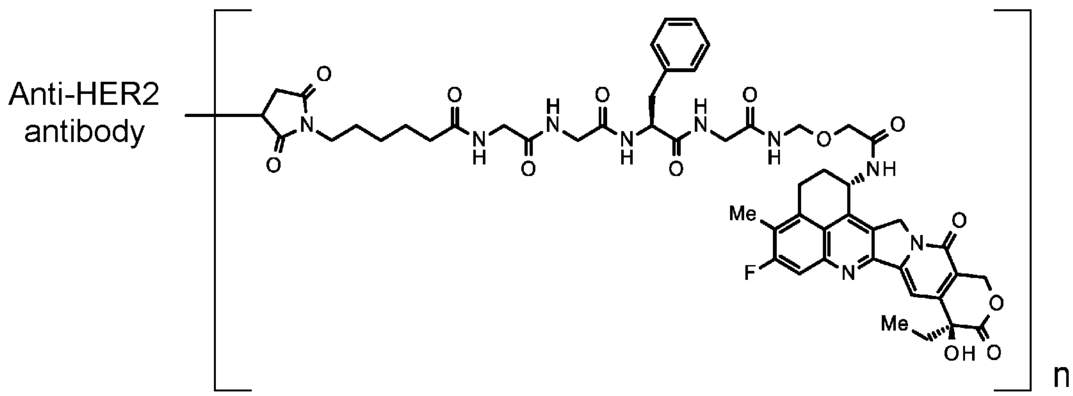

wherein a drug-linker is conjugated to the anti-HER2 antibody via a thioether bond; and n represents the average number of units of the drug-linker conjugated per antibody molecule, wherein n is in the range of from 7 to 8.
[57] The anti-HER2 antibody-drug conjugate according to any one of [51] to [56], wherein the anti-HER2 antibody-drug conjugate is trastuzumab deruxtecan.

[58] The anti-HER2 antibody-drug conjugate according to any one of [51] to [57], wherein the HER dimerization inhibitor is an antibody capable of binding to subdomain II of an extracellular domain of HER2 protein.

[59] The anti-HER2 antibody-drug conjugate according to any one of [51] to [57], wherein the HER dimerization inhibitor is pertuzumab.

[60] The anti-HER2 antibody-drug conjugate according to any one of [51] to [59], wherein the anti-HER2 antibody-drug conjugate and the HER dimerization inhibitor are separately contained as active components in different formulations, and are administered simultaneously or at different times.

[61] The anti-HER2 antibody-drug conjugate according to any one of [51] to [59], wherein the anti-HER2 antibody-drug conjugate and the HER dimerization inhibitor are contained as active components in a single formulation and administered.

[62] The anti-HER2 antibody-drug conjugate according to any one of [51] to [61], wherein the anti-HER2 antibody-drug conjugate is for use in treating cancer.

[63] The anti-HER2 antibody-drug conjugate according to [62], wherein the cancer is at least one selected from the group consisting of breast cancer, gastric cancer, colorectal cancer, lung cancer, esophageal cancer, head-and-neck cancer, gastroesophageal junction adenocarcinoma, biliary tract cancer, Paget's disease, pancreatic cancer, ovarian cancer, uterine carcinosarcoma, bladder cancer, prostate cancer, urothelial cancer, gastrointestinal stromal tumor, uterine cervix cancer, squamous cell carcinoma, peritoneal cancer, liver cancer, hepatocellular cancer, endometrial cancer, kidney cancer, vulval cancer, thyroid cancer, penis cancer, leukemia, malignant lymphoma, plasmacytoma, myeloma, glioblastoma multiforme, sarcoma, osteosarcoma, and melanoma.

[64] The anti-HER2 antibody-drug conjugate according to [62], wherein the cancer is at least one selected from the group consisting of breast cancer, gastric cancer, colorectal cancer, lung cancer, esophageal cancer, head-and-neck cancer, gastroesophageal junction adenocarcinoma, biliary tract cancer, Paget's disease, pancreatic cancer, ovarian cancer, uterine carcinosarcoma, bladder cancer, and prostate cancer.

[65] The anti-HER2 antibody-drug conjugate according to [62], wherein the cancer is breast cancer.

[66] The anti-HER2 antibody-drug conjugate according to any one of [62] to [65], wherein the cancer is HER2-overexpressing cancer.

[67] The anti-HER2 antibody-drug conjugate according to [66], wherein the HER2-overexpressing cancer is cancer given a score of 3+ for the expression of HER2 in an immunohistochemical method.

[68] The anti-HER2 antibody-drug conjugate according to [66], wherein the HER2-overexpressing cancer is cancer given a score of 2+ for the expression of HER2 in an immunohistochemical method and determined as positive for the expression of HER2 in an *in situ* hybridization method.

[69] The anti-HER2 antibody-drug conjugate according to any one of [62] to [65], wherein the cancer is HER2 low-expressing cancer.

[70] The anti-HER2 antibody-drug conjugate according to [69], wherein the HER2 low-expressing cancer is cancer given a score of 2+ for the expression of HER2 in an immunohistochemical method and determined as negative for the expression of HER2 in an *in situ* hybridization method.

[71] The anti-HER2 antibody-drug conjugate according to [69], wherein the HER2 low-expressing cancer is cancer given a score of 1+ for the expression of HER2 in an immunohistochemical method.

[72] The anti-HER2 antibody-drug conjugate according to [69], wherein the HER2 low-expressing cancer is cancer given a score of >0 and <1+ for the expression of HER2 in an immunohistochemical method.

[73] The anti-HER2 antibody-drug conjugate according to any one of [62] to [72], which exerts a significantly superior antitumor effect than single administration of the anti-HER2 antibody-drug conjugate.

[74] The anti-HER2 antibody-drug conjugate according to any one of [62] to [73], wherein the anti-HER2 antibody-drug conjugate is for use in treating cancer untreatable with single administration of the anti-HER2 antibody-drug conjugate.

[75] The anti-HER2 antibody-drug conjugate according to any one of [62] to [74], wherein the anti-HER2 antibody-drug conjugate and the HER dimerization inhibitor exert a synergistic antitumor effect.

[76] Use of an anti-HER2 antibody-drug conjugate for the manufacture of a medicament for treating a disease through being administered in combination with a HER dimerization inhibitor, wherein a drug-linker represented by the following formula:

[Formula 7]

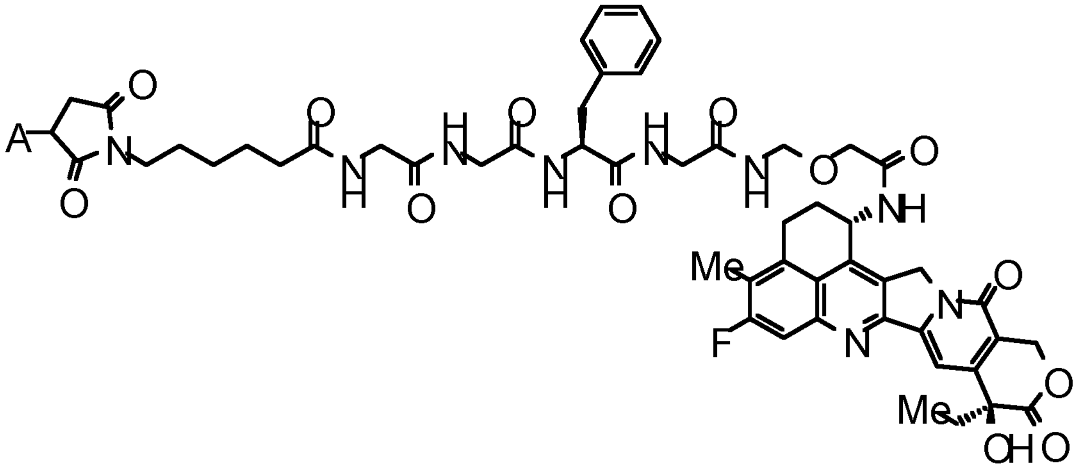

wherein A represents a connecting position to an anti-HER2 antibody,
is conjugated to the anti-HER2 antibody via a thioether bond in the anti-HER2 antibody-drug conjugate.

[77] The use according to [76], wherein the anti-HER2 antibody is an antibody comprising a heavy chain comprising CDRH1 consisting of an amino acid sequence represented by SEQ ID NO: 3, CDRH2 consisting of an amino acid sequence represented by SEQ ID NO: 4, and CDRH3 consisting of an amino acid sequence represented by SEQ ID NO: 5, and a light chain comprising CDRL1 consisting of an amino acid sequence represented by SEQ ID NO: 6, CDRL2 consisting of an amino acid sequence consisting of amino acid residues 1 to 3 of SEQ ID NO: 7, and CDRL3 consisting of an amino acid sequence represented by SEQ ID NO: 8.
[78] The use according to [76], wherein the anti-HER2 antibody is an antibody comprising a heavy chain comprising a heavy chain variable region consisting of an amino acid sequence represented by SEQ ID NO: 9, and a light chain comprising a light chain variable region consisting of an amino acid sequence represented by SEQ ID NO: 10.
[79] The use according to [76], wherein the anti-HER2 antibody is an antibody comprising a heavy chain consisting of an amino acid sequence represented by SEQ ID NO: 1 and a light chain consisting of an amino acid sequence represented by SEQ ID NO: 2.
[80] The use according to [76], wherein the anti-HER2 antibody is an antibody comprising a heavy chain consisting of an amino acid sequence represented by SEQ ID NO: 11 and a light chain consisting of an amino acid sequence represented by SEQ ID NO: 2.
[81] The use according to any one of [76] to [80], wherein the anti-HER2 antibody-drug conjugate is an anti-HER2 antibody-drug conjugate represented by the following formula:

[Formula 8]

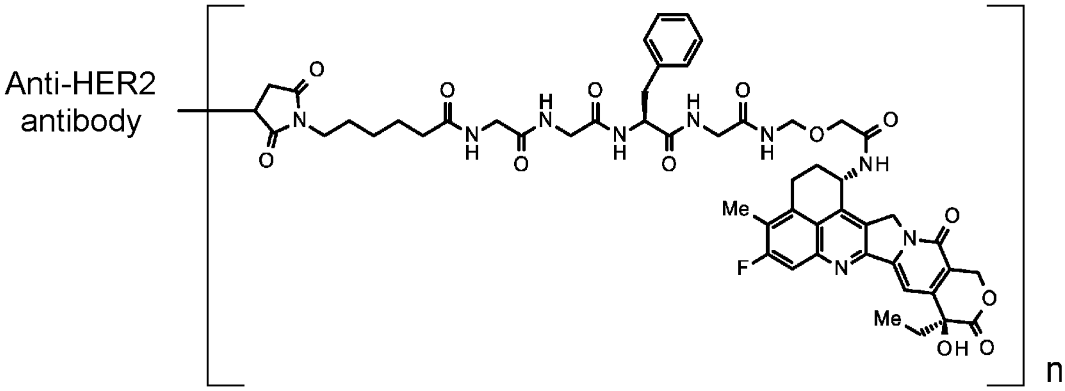

wherein a drug-linker is conjugated to the anti-HER2 antibody via a thioether bond; and n represents the average number of units of the drug-linker conjugated per antibody molecule, wherein n is in the range of from 7 to 8.

[82] The use according to any one of [76] to [81], wherein the anti-HER2 antibody-drug conjugate is trastuzumab deruxtecan.

[83] The use according to any one of [76] to [82], wherein the HER dimerization inhibitor is an antibody capable of binding to subdomain II of an extracellular domain of HER2 protein.

[84] The use according to any one of [76] to [82], wherein the HER dimerization inhibitor is pertuzumab.

[85] The use according to any one of [76] to [84], wherein the anti-HER2 antibody-drug conjugate and the HER dimerization inhibitor are separately contained as active components in different formulations, and are administered simultaneously or at different times.

[86] The use according to any one of [76] to [84], wherein the anti-HER2 antibody-drug conjugate and the HER dimerization inhibitor are contained as active components in a single formulation and administered.

[87] The use according to any one of [76] to [86], wherein the use is for treating cancer.

[88] The use according to [87], wherein the cancer is at least one selected from the group consisting of breast cancer, gastric cancer, colorectal cancer, lung cancer, esophageal cancer, head-and-neck cancer, gastroesophageal junction adenocarcinoma, biliary tract cancer, Paget's disease, pancreatic cancer, ovarian cancer, uterine carcinosarcoma, bladder cancer, prostate cancer, urothelial cancer, gastrointestinal stromal tumor, uterine cervix cancer, squamous cell carcinoma, peritoneal cancer, liver cancer, hepatocellular cancer, endometrial cancer, kidney cancer, vulval cancer, thyroid cancer, penis cancer, leukemia, malignant lymphoma, plasmacytoma, myeloma, glioblastoma multiforme, sarcoma, osteosarcoma, and melanoma.

[89] The use according to [87], wherein the cancer is at least one selected from the group consisting of breast cancer, gastric cancer, colorectal cancer, lung cancer, esophageal cancer, head-and-neck cancer, gastroesophageal junction adenocarcinoma, biliary tract cancer, Paget's disease, pancreatic cancer, ovarian cancer, uterine carcinosarcoma, bladder cancer, and prostate cancer.

[90] The use according to [87], wherein the cancer is breast cancer.

[91] The use according to any one of [87] to [90], wherein the cancer is HER2-overexpressing cancer.

[92] The use according to [91], wherein the HER2-overexpressing cancer is cancer given a score of 3+ for the expression of HER2 in an immunohistochemical method.

[93] The use according to [91], wherein the HER2-overexpressing cancer is cancer given a score of 2+ for the expression of HER2 in an immunohistochemical method and determined as positive for the expression of HER2 in an *in situ* hybridization method.

[94] The use according to any one of [87] to [90], wherein the cancer is HER2 low-expressing cancer.

[95] The cancer according to [94], wherein the HER2 low-expressing cancer is cancer given a score of 2+ for the expression of HER2 in an immunohistochemical method and determined as negative for the expression of HER2 in an *in situ* hybridization method.

[96] The use according to [94], wherein the HER2 low-expressing cancer is cancer given a score of 1+ for the expression of HER2 in an immunohistochemical method.

[97] The use according to [94], wherein the HER2 low-expressing cancer is cancer given a score of >0 and <1+ for the expression of HER2 in an immunohistochemical method.

[98] The use according to any one of [76] to [97], which exerts a significantly superior antitumor effect than single administration of the anti-HER2 antibody-drug conjugate.

[99] The use according to any one of [76] to [98], wherein the anti-HER2 antibody-drug conjugate is for use in treating cancer untreatable with single administration of the anti-HER2 antibody-drug conjugate.

[100] The use according to any one of [76] to [99], wherein the anti-HER2 antibody-drug conjugate and the HER

dimerization inhibitor exert a synergistic antitumor effect.

Advantageous Effects of Invention

The present invention provides a pharmaceutical composition, wherein a specific anti-HER2 antibody-drug conjugate and a HER dimerization inhibitor are administered in combination, and/or a method of treatment, comprising administering a specific anti-HER2 antibody-drug conjugate and a HER dimerization inhibitor in combination to a subject.

Brief Description of Drawings

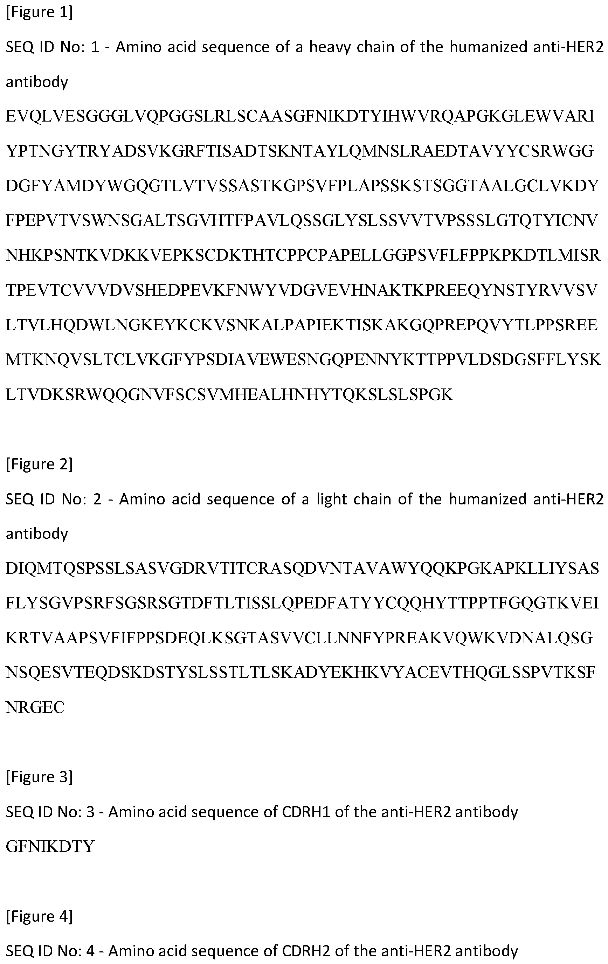

[Figure 1] Figure 1 shows an amino acid sequence of a heavy chain of an anti-HER2 antibody (SEQ ID NO: 1).
[Figure 2] Figure 2 shows an amino acid sequence of a light chain of an anti-HER2 antibody (SEQ ID NO: 2).
[Figure 3] Figure 3 shows an amino acid sequence of CDRH1 of an anti-HER2 antibody (SEQ ID NO: 3).
[Figure 4] Figure 4 shows an amino acid sequence of CDRH2 of an anti-HER2 antibody (SEQ ID NO: 4).

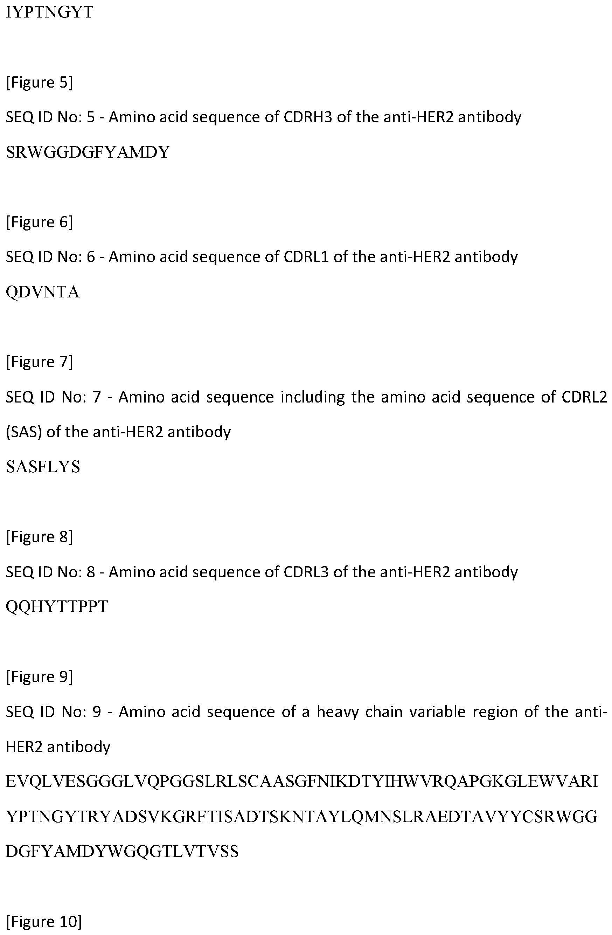

[Figure 5] Figure 5 shows an amino acid sequence of CDRH3 of an anti-HER2 antibody (SEQ ID NO: 5).
[Figure 6] Figure 6 shows an amino acid sequence of CDRL1 of an anti-HER2 antibody (SEQ ID NO: 6).
[Figure 7] Figure 7 shows an amino acid sequence including an amino acid sequence of CDRL2 (SAS) of an anti-HER2 antibody (SEQ ID NO: 7).
[Figure 8] Figure 8 shows an amino acid sequence of CDRL3 of an anti-HER2 antibody (SEQ ID NO: 8).
[Figure 9] Figure 9 shows an amino acid sequence of a heavy chain variable region of an anti-HER2 antibody (SEQ ID NO: 9).
[Figure 10] Figure 10 shows an amino acid sequence of a light chain variable region of an anti-HER2 antibody (SEQ ID NO: 10).
[Figure 11] Figure 11 shows an amino acid sequence of a heavy chain of an anti-HER2 antibody (SEQ ID NO: 11).
[Figure 12] Figure 12 shows an amino acid sequence of HER2 protein (SEQ ID NO: 12).
[Figure 13] Figure 13 is a diagram showing tumor growth suppressing effects in mice with subcutaneously transplanted KPL4 cells on single administration groups of HER2-ADC (1) and pertuzumab respectively, and a combined administration group of HER2-ADC (1) and pertuzumab.
[Figure 14] Figure 14 is a diagram showing tumor growth suppressing effects in mice with subcutaneously transplanted MDA-MB-453 cells on single administration groups of HER2-ADC (1) and pertuzumab respectively, and a combined administration group of HER2-ADC (1) and pertuzumab.

Description of Embodiments

Hereinafter, preferred modes for carrying out the present invention are described. The embodiments described below are given merely for illustrating one example of a typical embodiment of the present invention and are not intended to limit the scope of the present invention.

1. Definition

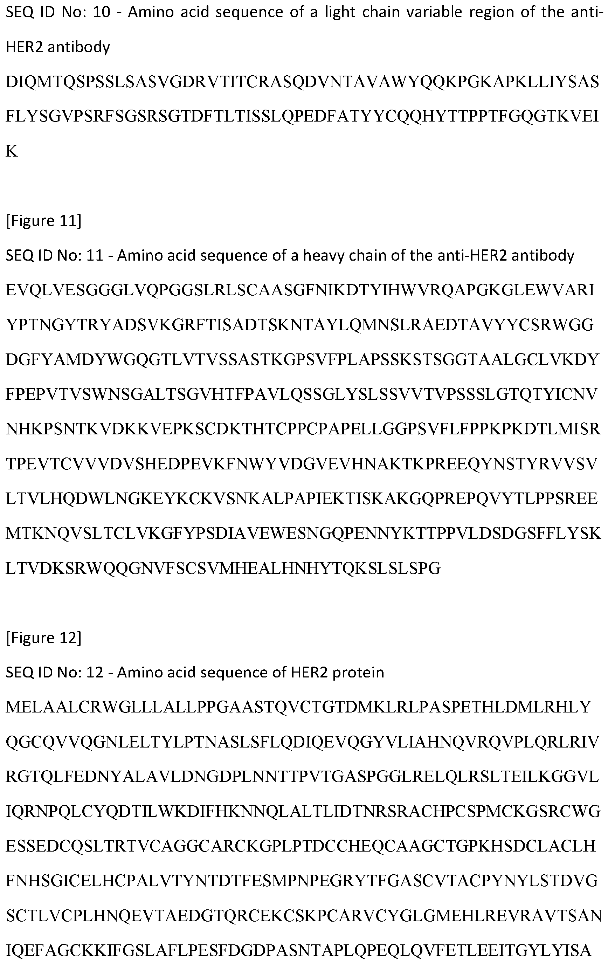

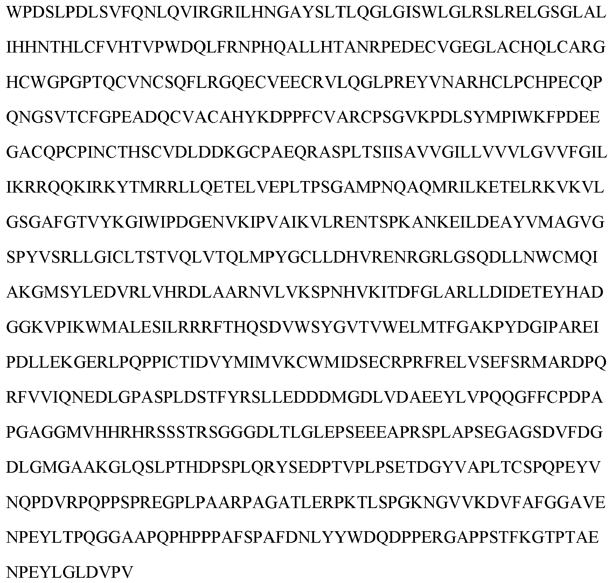

In the present invention, "HER2", the meaning of which is the same as that of human epidermal growth factor receptor 2 (may also be called neu or ErbB-2), is a transmembrane receptor belonging to the epidermal growth factor receptor (EGFR) subfamily of receptor tyrosine kinases together with HER1 (EGFR, ErbB-1), HER3 (ErbB-3), and HER4 (ErbB-4). HER2 is known to play an important role in growth, differentiation, and/or survival of cells in normal cells and tumor cells through activation due to undergoing autophosphorylation of an intracellular tyrosine residue by heterodimer formation with HER1, HER3, or HER4.
In the present invention, the term "HER2 protein" is used synonymously with HER2. The expression of HER2 protein can be detected by using a method well known to those skilled in the art such as an immunohistochemistry (IHC) method.
SEQ ID NO: 12 (Figure 12) represents the amino acid sequence of HER2 protein. In SEQ ID NO: 12, the amino acid sequence consisting of amino acid residues 1 to 652 is referred to as the "extracellular domain of HER2 protein", the amino acid sequence consisting of amino acid residues 653 to 675 as the "transmembrane domain of HER2 protein", and the amino acid sequence consisting of amino acid residues 676 to 1255 as the "intracellular domain of HER2 protein".
In the present invention, "subdomain II of the extracellular domain of HER2 protein" corresponds to the amino acid sequence consisting of amino acid residues 162 to 342 of SEQ ID NO: 12.
In the present invention, "subdomain IV of the extracellular domain of HER2 protein" corresponds to the amino

acid sequence consisting of amino acid residues 475 to 641 of SEQ ID NO: 12.

In the present invention the meaning of the term "HER2 gene" is the same as that of the term human epidermal growth factor receptor 2-related oncogene. HER2 protein is one of gene products of the HER2 gene.

SEQ ID NO: 13 represents the nucleotide sequence of the HER2 gene (cDNA).

In the present invention, the term "anti-HER2 antibody" refers to an antibody which binds specifically to HER2 and preferably has an activity of internalization in HER2-expressing cells by binding to HER2, in other words, an antibody which has an activity of binding to HER2 and then moving into HER2-expressing cells.

In the present invention, the terms "cancer" and "tumor" are synonymously used.

2. Anti-HER2 antibody-drug conjugate

The anti-HER2 antibody-drug conjugate used in the present invention is an anti-HER2 antibody-drug conjugate in which a drug-linker represented by the following formula:

[Formula 9]

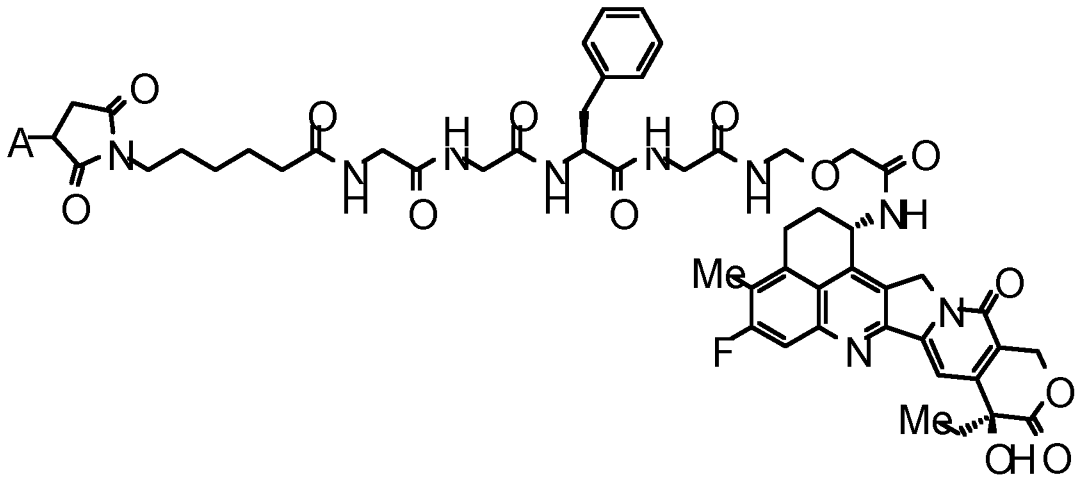

wherein A represents a connecting position to an anti-HER2 antibody,
is conjugated to the anti-HER2 antibody via a thioether bond.

In the present invention, the partial structure consisting of a linker and a drug in the anti-HER2 antibody-drug conjugate is referred to as a "drug-linker". The drug-linker is connected to a thiol group (in other words, the sulfur atom of a cysteine residue) formed at an interchain disulfide bond site (two sites between heavy chains, and two sites between a heavy chain and a light chain) in the antibody.

The drug-linker of the present invention includes exatecan (IUPAC name: (1S,9S)-1-amino-9-ethyl-5-fluoro-1,2,3,9,12,15-hexahydro-9-hydroxy-4-methyl-10H,13H-benzo[de]pyrano[3',4':6,7]indolizino[1,2-b]quinolin-10,13-dione, (also expressed as chemical name: (1S,9S)-1-amino-9-ethyl-5-fluoro-2,3-dihydro-9-hydroxy-4-methyl-1H,12H-benzo[de]pyrano[3',4':6,7]indolizino[1,2-b]quinolin-10,13(9H,15H)-dione)), which is a topoisomerase I inhibitor, as a component. Exatecan is a camptothecin derivative having an antitumor effect, represented by the following formula:

[Formula 10]

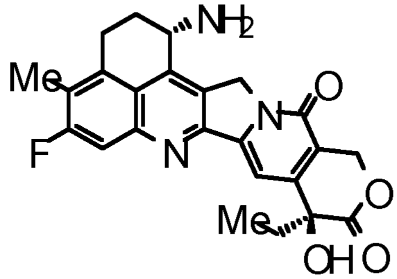

The anti-HER2 antibody-drug conjugate used in the present invention can also be represented by the following formula:

[Formula 11]

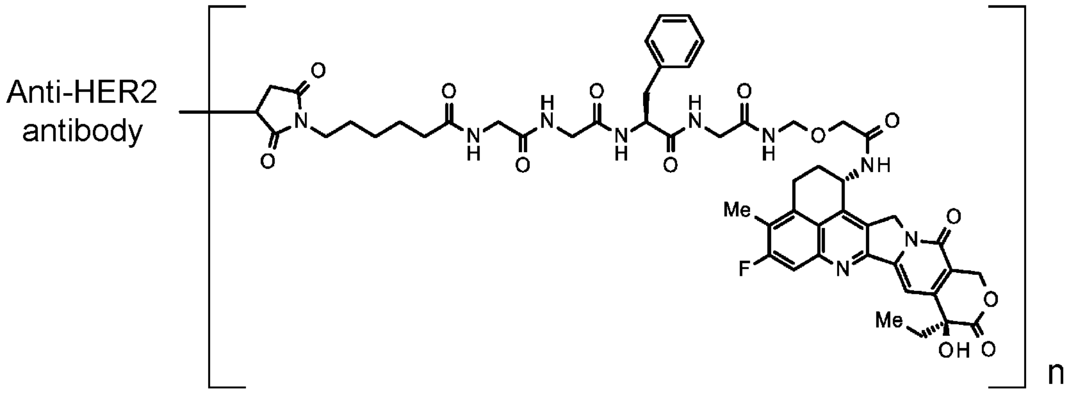

wherein, the drug-linker is conjugated to an anti-HER2 antibody via a thioether bond. The meaning of n is the same as that of what is called the average number of conjugated drug molecules (DAR; Drug-to-Antibody Ratio), and indicates the average number of units of the drug-linker conjugated per antibody molecule.

The average number of units of the drug-linker conjugated per antibody molecule in the anti-HER2 antibody-drug conjugate used in the present invention is preferably 2 to 8, more preferably 3 to 8, even more preferably 7 to 8, even more preferably 7.5 to 8, and even more preferably about 8.

After migrating into tumor cells, the anti-HER2 antibody-drug conjugate used in the present invention is cleaved at the linker portion to release the compound represented by the following formula:

[Formula 12]

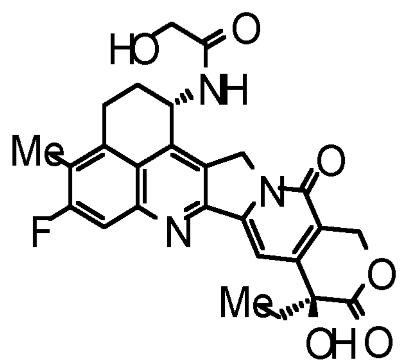

The aforementioned compound is inferred to be the original source of the antitumor activity of the aforementioned anti-HER2 antibody-drug conjugate used in the present invention, and has been confirmed to have a topoisomerase I inhibitory effect (Ogitani Y. et al., Clinical Cancer Research, 2016, Oct 15; 22(20): 5097-5108, Epub 2016 Mar 29).

The anti-HER2 antibody-drug conjugate used in the present invention is also known to have a bystander effect (Ogitani Y. et al., Cancer Science (2016) 107, 1039-1046). The bystander effect is exerted through a process such that the anti-HER2 antibody-drug conjugate used in the present invention is internalized in cancer cells expressing the target, and the aforementioned compound exerts an antitumor effect also on cancer cells which are present therearound and not expressing the target.

The anti-HER2 antibody-drug conjugate used in the present invention can be produced with reference to descriptions in International Publication No. WO 2015/115091 and so on.

The anti-HER2 antibody in the anti-HER2 antibody-drug conjugate used in the present invention is preferably an antibody comprising a heavy chain comprising CDRH1 consisting of an amino acid sequence represented by SEQ

ID NO: 3 (an amino acid sequence consisting of amino acid residues 26 to 33 of SEQ ID NO: 1), CDRH2 consisting of an amino acid sequence represented by SEQ ID NO: 4 (an amino acid sequence consisting of amino acid residues 51 to 58 of SEQ ID NO: 1), and CDRH3 consisting of an amino acid sequence represented by SEQ ID NO: 5 (an amino acid sequence consisting of amino acid residues 97 to 109 of SEQ ID NO: 1), and a light chain comprising CDRL1 consisting of an amino acid sequence represented by SEQ ID NO: 6 (an amino acid sequence consisting of amino acid residues 27 to 32 of SEQ ID NO: 2), CDRL2 consisting of an amino acid sequence consisting of amino acid residues 1 to 3 of SEQ ID NO: 7 (an amino acid sequence consisting of amino acid residues 50 to 52 of SEQ ID NO: 2), and CDRL3 consisting of an amino acid sequence represented by SEQ ID NO: 8 (an amino acid sequence consisting of amino acid residues 89 to 97 of SEQ ID NO: 2),

more preferably an antibody comprising a heavy chain comprising a heavy chain variable region consisting of an amino acid sequence represented by SEQ ID NO: 9 (an amino acid sequence consisting of amino acid residues 1 to 120 of SEQ ID NO: 1) and a light chain comprising a light chain variable region consisting of an amino acid sequence represented by SEQ ID NO: 10 (an amino acid sequence consisting of amino acid residues 1 to 107 of SEQ ID NO: 2), and
even more preferably an antibody comprising a heavy chain consisting of an amino acid sequence represented by SEQ ID NO: 1 and a light chain consisting of an amino acid sequence represented by SEQ ID NO: 2, or an antibody comprising a heavy chain consisting of an amino acid sequence represented by SEQ ID NO: 11 (an amino acid sequence consisting of amino acid residues 1 to 449 of SEQ ID NO: 1) and a light chain consisting of an amino acid sequence represented by SEQ ID NO: 2.

The anti-HER2 antibody-drug conjugate used in the present invention is preferably trastuzumab deruxtecan.

3. Production of the anti-HER2 antibody

The HER2 protein used in the present invention can be applied through direct purification from HER2-expressing cells of a human or a non-human mammal (a rat, a mouse, etc.), or through preparation of a cell membrane fraction of the cells. Alternatively, the HER2 protein can be obtained by *in vitro* synthesis of HER2, or by genetically engineering host cells to produce HER2. In the gene engineering, specifically, the protein can be obtained by incorporating HER2 cDNA into a vector capable of expressing it and then synthesizing the protein in a solution containing enzymes, substrates, and energetic materials necessary for transcription and translation, or by transforming host cells of another prokaryote or eukaryote to allow the host cells to express HER2. In addition, the above-described genetically engineered HER2-expressing cells or a cell line expressing HER2 can be used as the HER2 protein.
The DNA sequence for and the amino acid sequence of HER2 are available in public databases, and, for example, accessible by accession numbers of M11730 (Genbank), NP_004439.2 (NCBI), and so on.
Furthermore, HER2 includes a protein consisting of an amino acid sequence where 1 or several amino acid substitution, deletion, addition, and/or insertion has been made to the amino acid sequence of HER2, but keeping a biological activity equivalent to the protein.
Human HER2 protein is composed of a signal sequence consisting of 22 amino acid residues at the N terminus, an extracellular domain consisting of 630 amino acid residues, a transmembrane domain consisting of 23 amino acid residues, and an intracellular domain consisting of 580 amino acid residues.
The anti-HER2 antibody used in the present invention can be obtained by a procedure known in the art. For example, the anti-HER2 antibody can be obtained using a method usually carried out in the art, which involves immunizing animals with antigenic HER2 or any polypeptide selected from the amino acid sequence of HER2 and collecting and purifying antibodies produced *in vivo*. The origin of the antigen is not limited to humans, and the animals may be immunized with an antigen derived from a non-human animal such as a mouse, a rat and the like. In this case, the cross-reactivity of antibodies binding to the obtained heterologous antigen with human antigens can be tested to screen for an anti-HER2 antibody applicable to a human disease.
Alternatively, antibody-producing cells which produce antibodies against the antigen can be fused with myeloma cells according to a method known in the art (for example, Kohler and Milstein, Nature (1975) 256, p.495-497; Kennet, R. ed., Monoclonal Antibodies, p.365-367, Plenum Press, N.Y. (1980)), to establish hybridomas, from which monoclonal antibodies can in turn be obtained.
The antigen can be obtained by genetically engineering host cells to produce a gene encoding the antigenic protein. Specifically, vectors that permit expression of the antigen gene are prepared and transferred to host cells so that the gene is expressed. The antigen thus expressed can be purified. The antibody can also be obtained by a method of immunizing animals with the above-described genetically engineered antigen-expressing cells or a cell line expressing the antigen.
The anti-HER2 antibody used in the present invention is preferably a recombinant antibody obtained by artificial

modification for the purpose of decreasing heterologous antigenicity to humans such as a chimeric antibody or a humanized antibody, or is preferably an antibody having only the gene sequence of an antibody derived from a human, that is, a human antibody. These antibodies can be produced using a known method.

As the chimeric antibody, an antibody in which antibody variable and constant regions are derived from different species, for example, a chimeric antibody in which a mouse- or rat-derived antibody variable region is connected to a human-derived antibody constant region can be exemplified (Proc. Natl. Acad. Sci. USA, 81, 6851-6855, (1984)).

As the humanized antibody, an antibody obtained by integrating only the complementarity determining region (CDR) of a heterologous antibody into a human-derived antibody (Nature (1986) 321, pp. 522-525), an antibody obtained by grafting a part of the amino acid residues of the framework of a heterologous antibody as well as the CDR sequence of the heterologous antibody to a human antibody by a CDR-grafting method (WO 90/07861), and an antibody humanized using a gene conversion mutagenesis strategy (U.S. Patent No. 5821337) can be exemplified.

As the human antibody, an antibody generated by using a human antibody-producing mouse having a human chromosome fragment including genes of a heavy chain and light chain of a human antibody (see Tomizuka, K. et al., Nature Genetics (1997) 16, p.133-143; Kuroiwa, Y. et. al., Nucl. Acids Res. (1998) 26, p.3447-3448; Yoshida, H. et. al., Animal Cell Technology: Basic and Applied Aspects vol.10, p.69-73 (Kitagawa, Y., Matsuda, T. and Iijima, S. eds.), Kluwer Academic Publishers, 1999; Tomizuka, K. et. al., Proc. Natl. Acad. Sci. USA (2000) 97, p.722-727, etc.) can be exemplified. As an alternative, an antibody obtained by phage display, the antibody being selected from a human antibody library (see Wormstone, I. M. et. al, Investigative Ophthalmology & Visual Science. (2002) 43 (7), p.2301-2308; Carmen, S. et. al., Briefings in Functional Genomics and Proteomics (2002), 1 (2), p.189-203; Siriwardena, D. et. al., Ophthalmology (2002) 109 (3), p.427-431, etc.) can be exemplified.

In the anti-HER2 antibody used in the present invention, modified variants of the antibody are also included. The modified variant refers to a variant obtained by subjecting the antibody according to the present invention to chemical or biological modification. Examples of the chemically modified variant include variants including a linkage of a chemical moiety to an amino acid skeleton, variants including a linkage of a chemical moiety to an N-linked or O-linked carbohydrate chain, etc. Examples of the biologically modified variant include variants obtained by post-translational modification (such as N-linked or O-linked glycosylation, N- or C-terminal processing, deamidation, isomerization of aspartic acid, or oxidation of methionine), and variants in which a methionine residue has been added to the N terminus by being expressed in a prokaryotic host cell. Further, an antibody labeled so as to enable the detection or isolation of the anti-HER2 antibody or an antigen used in the present invention, for example, an enzyme-labeled antibody, a fluorescence-labeled antibody, and an affinity-labeled antibody are also included in the meaning of the modified variant. Such a modified variant of the anti-HER2 antibody used in the present invention is useful for improving the stability and blood retention of the antibody, reducing the antigenicity thereof, detecting or isolating an antibody or an antigen, and so on.

Further, by regulating the modification of a glycan which is linked to the anti-HER2 antibody used in the present invention (glycosylation, defucosylation, etc.), it is possible to enhance antibody-dependent cellular cytotoxic activity. As the technique for regulating the modification of a glycan of antibodies, International Publication No. WO 99/54342, International Publication No. WO 00/61739, International Publication No. WO 02/31140, etc. are known. However, the technique is not limited thereto. In the anti-HER2 antibody used in the present invention, antibodies in which the modification of a glycan is regulated are also included.

It is known that a lysine residue at the carboxyl terminus of the heavy chain of an antibody produced in a cultured mammalian cell is deleted (Journal of Chromatography A, 705: 129-134 (1995)), and it is also known that two amino acid residues (glycine and lysine) at the carboxyl terminus of the heavy chain of an antibody produced in a cultured mammalian cell are deleted and a proline residue newly located at the carboxyl terminus is amidated (Analytical Biochemistry, 360: 75-83 (2007)). However, such deletion and modification of the heavy chain sequence do not affect the antigen-binding affinity and the effector function (complement activation, antibody-dependent cellular cytotoxicity, etc.) of the antibody. Therefore, in the anti-HER2 antibody used in the present invention, antibodies subjected to such modification and functional fragments of the antibody are also included, and deletion variants in which one or two amino acids have been deleted at the carboxyl terminus of the heavy chain, variants obtained by amidation of the deletion variants (for example, a heavy chain in which the carboxyl terminal proline residue has been amidated), and the like are also included. The type of deletion variant having a deletion at the carboxyl terminus of the heavy chain of the anti-HER2 antibody used in the present invention is not limited to the above variants as long as the antigen-binding affinity and the effector function are conserved. The two heavy chains constituting the anti-HER2 antibody used in the present invention may be of one type selected from the group consisting of a full-length heavy chain and the above-described deletion variant, or may be of two types in combination selected therefrom. The ratio of the amount of each deletion variant can be affected by the type of cultured mammalian cells which produce the anti-HER2 antibody used in the present invention and the culture conditions; however, an antibody in which one amino acid residue at the carboxyl terminus has been deleted in both of the two heavy chains in the anti-HER2 antibody used in the present invention can be preferably exemplified.

As isotypes of the anti-HER2 antibody used in the present invention, for example, IgG (IgG1, IgG2, IgG3, IgG4)

can be exemplified. Preferably, IgG1 or IgG2 can be exemplified. Variants of them can be used as the anti-HER2 antibody according to the present invention.

4. Production of the anti-HER2 antibody-drug conjugate

A drug-linker intermediate for use in the production of the anti-HER2 antibody-drug conjugate according to the present invention is represented by the following formula.

[Formula 13]

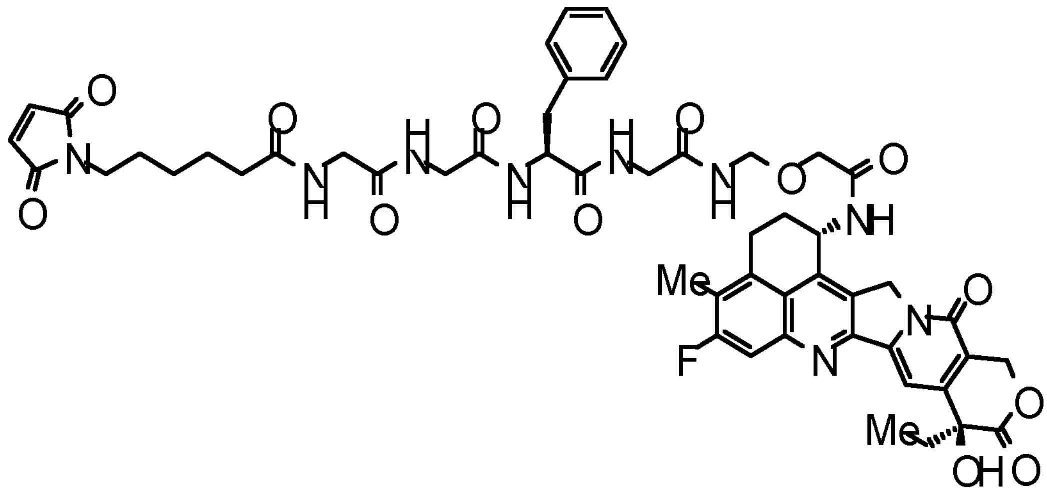

The drug-linker intermediate can be expressed as the chemical name N-[6-(2,5-dioxo-2,5-dihydro-1H-pyrrol-1-yl)hexanoyl]glycylglycyl-L-phenylalanyl-N-[(2-{[(1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl]amino}-2-oxoethoxy)methyl]glycinamide, and can be produced with reference to descriptions in International Publication No. WO 2015/115091.

The anti-HER2 antibody-drug conjugate used in the present invention can be produced by reacting the above-described drug-linker intermediate and an anti-HER2 antibody having a thiol group (alternatively referred to as a sulfhydryl group).

The anti-HER2 antibody having a sulfhydryl group can be obtained by a method well known in the art (Hermanson, G. T, Bioconjugate Techniques, pp. 56-136, pp. 456-493, Academic Press (1996)). For example, by using 0.3 to 3 molar equivalents of a reducing agent such as tris(2-carboxyethyl)phosphine hydrochloride (TCEP) per interchain disulfide within the antibody and reacting with the anti-HER2 antibody in a buffer solution containing a chelating agent such as ethylenediamine tetraacetic acid (EDTA), an anti-HER2 antibody having a sulfhydryl group with partially or completely reduced interchain disulfides within the antibody can be obtained.

Further, by using 2 to 20 molar equivalents of the drug-linker intermediate per anti-HER2 antibody having a sulfhydryl group, an anti-HER2 antibody-drug conjugate in which 2 to 8 drug molecules are conjugated per antibody molecule can be produced.

The average number of conjugated drug molecules per antibody molecule of the anti-HER2 antibody-drug conjugate produced can be determined, for example, by a method of calculation based on measurement of UV absorbance for the anti-HER2 antibody-drug conjugate and the conjugation precursor thereof at two wavelengths of 280 nm and 370 nm (UV method), or a method of calculation based on quantification through HPLC measurement for fragments obtained by treating the antibody-drug conjugate with a reducing agent (HPLC method).

Conjugation between the anti-HER2 antibody and the drug-linker intermediate and calculation of the average number of conjugated drug molecules per antibody molecule of the anti-HER2 antibody-drug conjugate can be performed with reference to descriptions in International Publication No. WO 2015/115091 and so on.

5. HER dimerization inhibitor

In the present invention, the term "HER dimerization inhibitor" refers to a drug that binds to HER2 protein to inhibit the heterodimer formation of HER2 with HER1, HER3, or HER4. The HER dimerization inhibitor in the present invention is not limited as long as it is a drug having the function, and antibodies capable of binding to subdomain II of the extracellular domain of HER2 protein can be preferably exemplified. Further, pertuzumab can be more preferably

exemplified.

6. Medicament

Described in the following are a pharmaceutical composition and a method of treatment according to the present invention, wherein an anti-HER2 antibody-drug conjugate and a HER dimerization inhibitor are administered in combination.

The pharmaceutical composition and method of treatment of the present invention may be those in which the anti-HER2 antibody-drug conjugate and the HER dimerization inhibitor are separately contained as active components in different formulations and are administered simultaneously or at different times, or may be those in which the anti-HER2 antibody-drug conjugate and the HER dimerization inhibitor are contained as active components in a single formulation and administered.

The pharmaceutical composition and method of treatment of the present invention are characterized in that they exert a significantly superior antitumor effect than single administration of the anti-HER2 antibody-drug conjugate. This enables treatment of cancer untreatable with single administration of the anti-HER2 antibody-drug conjugate. Further, the pharmaceutical composition and method of treatment of the present invention are characterized in that they exert a significantly superior antitumor effect than single administration of the HER dimerization inhibitor. This enables treatment of cancer untreatable with single administration of the HER dimerization inhibitor. Furthermore, the pharmaceutical composition and method of treatment of the present invention are characterized in that the anti-HER2 antibody-drug conjugate and the HER dimerization inhibitor exert a synergistic antitumor effect.

The pharmaceutical composition and method of treatment of the present invention can be used for treating cancer, and can be preferably used for treating at least one disease selected from the group consisting of breast cancer (including triple-negative breast cancer and luminal breast cancer), gastric cancer (also called gastric adenocarcinoma), colorectal cancer (also called colon and rectal cancer, and including colon cancer and rectal cancer), lung cancer (including small cell lung cancer and non-small cell lung cancer), esophageal cancer, head-and-neck cancer (including salivary gland cancer and pharyngeal cancer), gastroesophageal junction adenocarcinoma, biliary tract cancer (including bile duct cancer), Paget’s disease, pancreatic cancer, ovarian cancer, uterine carcinosarcoma, bladder cancer, prostate cancer, urothelial cancer, gastrointestinal stromal tumor, uterine cervix cancer, squamous cell carcinoma, peritoneal cancer, liver cancer, hepatocellular cancer, endometrial cancer, kidney cancer, vulval cancer, thyroid cancer, penis cancer, leukemia, malignant lymphoma, plasmacytoma, myeloma, glioblastoma multiforme, sarcoma, osteosarcoma, and melanoma; and can more preferably be used for treating at least one cancer selected from the group consisting of breast cancer, gastric cancer, colorectal cancer, lung cancer, esophageal cancer, head-and-neck cancer, gastroesophageal junction adenocarcinoma, biliary tract cancer, Paget’s disease, pancreatic cancer, ovarian cancer, uterine carcinosarcoma, bladder cancer, and prostate cancer.

The pharmaceutical composition and method of treatment of the present invention can be preferably used for treatment of HER2-overexpressing cancer. The presence or absence of expression of HER2 can be checked by, for example, collecting tumor tissue from a cancer patient, and subjecting the formalin fixed paraffin embedded specimen (FFPE) to an examination at a gene product (protein) level, such as an immunohistochemistry (IHC) method, a flow cytometry, a western blot method, or an examination at a gene transcription level, such as an *in situ* hybridization method (ISH), a quantitative PCR method (q-PCR), or a microarray analysis; alternatively, it can also be checked by collecting cell-free blood circulating tumor DNA (ctDNA) from a cancer patient and subjecting to an examination which uses a method such as next-generation sequencing (NGS).

Further, the pharmaceutical composition and method of treatment of the present invention can be preferably used not only for HER2-overexpressing cancer but also for HER2 low-expressing cancer and HER2-mutated cancer.

In the present invention, the term "HER2-overexpressing cancer" is not particularly limited as long as it is recognized as HER2-overexpressing cancer by those skilled in the art. Preferred examples of the HER2-overexpressing cancer can include cancer given a score of 3+ for the expression of HER2 in an immunohistochemical method, and cancer given a score of 2+ for the expression of HER2 in an immunohistochemical method and determined as positive for the expression of HER2 in an *in situ* hybridization method. The *in situ* hybridization method of the present invention includes a fluorescence *in situ* hybridization method (FISH) and a dual color *in situ* hybridization method (DISH).

In the present invention, the term "HER2 low-expressing cancer" is not particularly limited as long as it is recognized as HER2 low-expressing cancer by those skilled in the art. Preferred examples of the HER2 low-expressing cancer can include cancer given a score of 2+ for the expression of HER2 in an immunohistochemical method and determined as negative for the expression of HER2 in an *in situ* hybridization method, cancer given a score of 1+ for the expression of HER2 in an immunohistochemical method, or cancer given a score of 0> and <1+ for the expression of HER2 in an immunohistochemical method.

The method for scoring the degree of HER2 expression by the immunohistochemical method, or the method for determining positivity or negativity to HER2 expression by the *in situ* hybridization method is not particularly limited

as long as it is recognized by those skilled in the art. Examples of the method can include a method described in the 4th edition of the guidelines for HER2 testing, breast cancer (developed by the Japanese Pathology Board for Optimal Use of HER2 for Breast Cancer).

The HER2 low-expressing cancer for which the pharmaceutical composition and method of treatment of the present invention can be used is preferably HER2 low-expressing breast cancer, HER2 low-expressing gastric cancer, HER2 low-expressing colorectal cancer, or HER2 low-expressing non-small cell lung cancer, and is more preferably HER2 low-expressing breast cancer.

In the present invention, the term "HER2-mutated cancer" refers to cancer with a mutation in the amino acid sequence of HER2 protein, or cancer with a mutation in the HER2 gene. Cancer including cancer cells with a HER2 mutation, even without the presence of HER2 mutations throughout tumor tissue, is categorized as HER2-mutated cancer (see International Publication No. WO 2019/230645, etc.).

The HER2-mutated cancer for which the pharmaceutical composition and method of treatment of the present invention can be used is preferably HER2-mutated non-small cell lung cancer, HER2-mutated gastric cancer, HER2-mutated breast cancer, or HER2-mutated colorectal cancer, and is more preferably HER2-mutated non-small cell lung cancer or HER2-mutated gastric cancer.

The pharmaceutical composition and method of treatment of the present invention can be preferably used for mammals, and can be more preferably used for humans.

The antitumor effect of the pharmaceutical composition and method of treatment of the present invention can be confirmed by, for example, generating a model in which cancer cells are transplanted to a test animal, and measuring reduction in tumor volume, life-prolonging effects due to applying the pharmaceutical composition and method of treatment of the present invention. Furthermore, comparison with the antitumor effect of single administration of each of the anti-HER2 antibody-drug conjugate and the HER dimerization inhibitor used in the present invention can provide confirmation of the combined effect of the anti-HER2 antibody-drug conjugate and the HER dimerization inhibitor used in the present invention.

In addition, the antitumor effect of the pharmaceutical composition and method of treatment of the present invention can be confirmed, in a clinical study, with the Response Evaluation Criteria in Solid Tumors (RECIST) evaluation method, WHO's evaluation method, Macdonald's evaluation method, measurement of body weight, and other methods; and can be determined by indicators such as Complete response (CR), Partial response (PR), Progressive disease (PD), Objective response rate (ORR), Duration of response (DoR), Progression-free survival (PFS), and Overall survival (OS).

The foregoing methods can provide confirmation of superiority in terms of the antitumor effect of the pharmaceutical composition and method of treatment of the present invention compared to existing pharmaceutical compositions and methods of treatment for cancer therapy.

The pharmaceutical composition and method of treatment of the present invention can retard growth of cancer cells, suppress their proliferation, and further can kill cancer cells. These effects can allow cancer patients to be free from symptoms caused by cancer or can achieve an improvement in the QOL of cancer patients and attain a therapeutic effect by sustaining the lives of the cancer patients. Even if the pharmaceutical composition and method of treatment do not accomplish the killing of cancer cells, they can achieve higher QOL of cancer patients while achieving longer-term survival, by inhibiting or controlling the growth of cancer cells.

The pharmaceutical composition of the present invention can be expected to exert a therapeutic effect by application as a systemic therapy to patients, and additionally, by local application to cancer tissues.

The pharmaceutical composition of the present invention may be administered as a pharmaceutical composition containing at least one pharmaceutically suitable ingredient. The pharmaceutically suitable ingredient can be suitably selected and applied from formulation additives or the like that are generally used in the art, in accordance with the dosage, administration, concentration or the like of the anti-HER2 antibody-drug conjugate used in the present invention and the HER dimerization inhibitor. For example, the anti-HER2 antibody-drug conjugate used in the present invention can be administered as a pharmaceutical composition containing a buffer such as a histidine buffer, an excipient such as sucrose or trehalose, and a surfactant such as Polysorbate 80. The pharmaceutical composition containing the anti-HER2 antibody-drug conjugate used in the present invention can be preferably used as an injection, can be more preferably used as an aqueous injection or a lyophilized injection, and can be even more preferably used as a lyophilized injection.

In the case that the pharmaceutical composition containing the anti-HER2 antibody-drug conjugate used in the present invention is an aqueous injection, it can be preferably diluted with a suitable diluent and then given as an intravenous infusion. For the diluent, a dextrose solution, physiological saline, and the like, can be exemplified, and a dextrose solution can be preferably exemplified, and a 5% dextrose solution can be more preferably exemplified.

In the case that the pharmaceutical composition containing the anti-HER2 antibody-drug conjugate used in the present invention is a lyophilized injection, it can be preferably dissolved in water for injection, subsequently a required amount can be diluted with a suitable diluent and then given as an intravenous infusion. For the diluent, a dextrose

solution, physiological saline, and the like, can be exemplified, and a dextrose solution can be preferably exemplified, and a 5% dextrose solution can be more preferably exemplified.

Examples of the administration route which may be used to administer the pharmaceutical composition of the present invention include intravenous, intradermal, subcutaneous, intramuscular, and intraperitoneal routes; and preferably include an intravenous route.

The anti-HER2 antibody-drug conjugate used in the present invention can be administered to a human once at intervals of 1 to 180 days, and can be preferably administered once a week, once every 2 weeks, once every 3 weeks, or once every 4 weeks, and can be even more preferably administered once every 3 weeks. Also, the anti-HER2 antibody-drug conjugate used in the present invention can be administered at a dose of about 0.001 to 100 mg/kg, and can be preferably administered at a dose of 0.8 to 12.4 mg/kg. The anti-HER2 antibody-drug conjugate used in the present invention can be more preferably administered once every 3 weeks at a dose of 0.8 mg/kg, 1.6 mg/kg, 3.2 mg/kg, 5.4 mg/kg, 6.4 mg/kg, 7.4 mg/kg, or 8 mg/kg, and can be even more preferably administered once every 3 weeks at a dose of 5.4 mg/kg or 6.4 mg/kg.

The HER dimerization inhibitor used in the present invention (preferably, pertuzumab) can be preferably administered to a human at a dose of 840 mg in the first administration, and thereafter once every 3 weeks at a dose of 420 mg.

The pharmaceutical composition and method of treatment of the present invention may further contain a cancer therapeutic agent other than the anti-HER2 antibody-drug conjugate and the HER dimerization inhibitor used in the present invention. The pharmaceutical composition and method of treatment of the present invention can also be administered in combination with another cancer therapeutic agent, thereby enhancing the antitumor effect. Other cancer therapeutic agents to be used for such purpose may be administered to a subject simultaneously, separately, or sequentially with the pharmaceutical composition of the present invention, or may be administered with varying each dosage interval. Such cancer therapeutic agents are not limited as long as they are agents having antitumor activity, and can be exemplified by at least one selected from the group consisting of irinotecan (CPT-11), cisplatin, carboplatin, oxaliplatin, fluorouracil (5-FU), gemcitabine, capecitabine, paclitaxel, docetaxel, doxorubicin, epirubicin, cyclophosphamide, mitomycin C, tegafur-gimeracil-oteracil combination, cetuximab, panitumumab, bevacizumab, ramucirumab, regorafenib, trifluridine-tipiracil combination, gefitinib, erlotinib, afatinib, methotrexate, pemetrexed, tamoxifen, toremifene, fulvestrant, leuprorelin, goserelin, letrozole, anastrozole, progesterone formulation, and lapatinib.

The pharmaceutical composition and method of treatment of the present invention can also be used in combination with radiotherapy. For example, a cancer patient may receive radiotherapy before and/or after or simultaneously with receiving therapy with the pharmaceutical composition of the present invention.

The pharmaceutical composition and method of treatment of the present invention can also be used as an adjuvant chemotherapy in combination with a surgical procedure. The pharmaceutical composition of the present invention may be administered for the purpose of diminishing the size of a tumor before a surgical procedure (referred to as pre-operative adjuvant chemotherapy or neoadjuvant therapy), or may be administered after a surgical procedure for the purpose of preventing the recurrence of a tumor (referred to as post-operative adjuvant chemotherapy or adjuvant therapy).

## Examples

The present invention is specifically described in view of the examples shown below. However, the present invention is not limited to these. Further, it is by no means to be interpreted in a limited way.

### Example 1: Production of the anti-HER2 antibody-drug conjugate

In accordance with a production method described in International Publication No. WO 2015/115091, by using a humanized anti-HER2 antibody (an antibody comprising a heavy chain consisting of an amino acid sequence represented by SEQ ID NO: 11 (an amino acid sequence consisting of amino acid residues 1 to 449 of SEQ ID NO: 1) and a light chain consisting of an amino acid sequence represented by SEQ ID NO: 2), an anti-HER2 antibody-drug conjugate in which a drug-linker represented by the following formula:

[Formula 14]

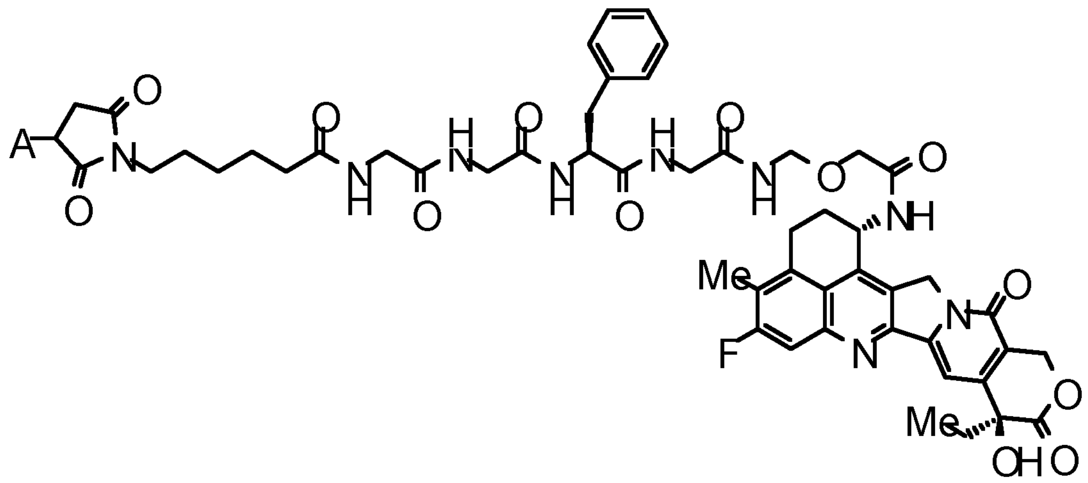

wherein A represents a connecting position to an anti-HER2 antibody,
is conjugated to the anti-HER2 antibody via a thioether bond (hereinafter referred to as the "HER2-ADC (1)") was produced. The DAR of the HER2-ADC (1) was 7.8.

Example 2: Antitumor study (1)

Mouse: Female 5-6-week-old BALB/c nude mice (CHARLES RIVER LABORATORIES JAPAN, INC.) were subjected to the experiments.

Measurement and calculation formula: In all studies, the major axis and minor axis of tumors were measured twice a week with an electronic digital caliper, and the tumor volume ($mm^3$) was calculated. The calculation formula is as shown below.

$$\text{Tumor volume } (mm^3) = 1/2 \times \text{Major axis (mm)} \times [\text{Minor axis (mm)}]^2$$

The HER2-ADC (1) was diluted with ABS buffer (10 mM acetate buffer [pH 5.5], 5% sorbitol), and intravenously administered in a fluid volume of 10 mL/kg to the tail vein. Pertuzumab was diluted with physiological saline, and intravenously administered in a fluid volume of 10 mL/kg to the tail vein.

Human breast cancer cell line KPL-4 [British Journal of Cancer, (1999) 79 (5/6). 707-717], which was obtained from Dr. Junichi Kurebayashi in Kawasaki Medical School, was suspended into physiological saline, subcutaneously transplanted at $1.5 \times 10^7$ cells into the right side of female nude mice, and the mice were randomly grouped 17 days after the transplantation (Day 0). The HER2-ADC (1) was intravenously administered to the tail vein at a dose of 7.5 mg/kg on Day 0. Pertuzumab was intravenously administered to the tail vein at a dose of 30 mg/kg on Day 0 and at a dose of 15 mg/kg on Day 7. Single administration groups of each drug, a combined administration group, and a solvent administration group as a control group were set up.

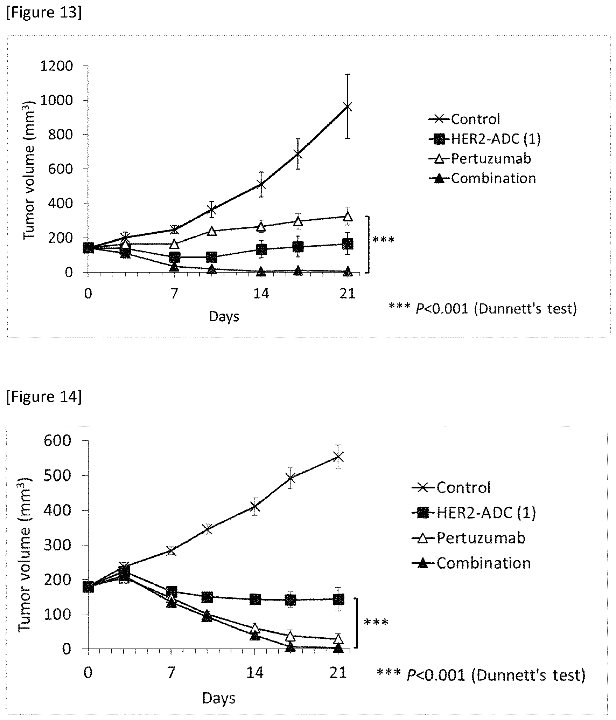

Results of a combination of HER2-ADC (1) and pertuzumab are shown in Figure 13. Single administration of pertuzumab showed a tumor growth inhibition (TGI) of 66% on the day of determination of effect, and loss of tumor was not found in any of six cases. Single administration of HER2-ADC (1) showed TGI of 83%, and loss of tumor was found in one of six cases. On the other hand, combined administration of HER2-ADC (1) and pertuzumab exhibited a significantly superior tumor growth suppression effect than single administration of pertuzumab ($P < 0.001$ [calculated by Dunnett's test; the same applies hereinafter]) and showed TGI of 100%, and loss of tumor was found in four of six cases. Here, in the Figure, the abscissa axis represents days after the first administration, and the longitudinal axis represents tumor volume. In addition, none of the single and combined administration groups exhibited any particular notable finding such as weight loss. Incidentally, in the following evaluation examples relating to antitumor studies, unless otherwise described, the studies are performed by the procedure used in this evaluation example.

Example 3: Antitumor study (2)

Human breast cancer cell line MDA-MB-453 cells (see Breast Cancer Res. 2011 Aug 12; 13(4): 215, and J Mol

Diagn. 2017 Mar; 19(2): 244-254, etc.), which were purchased from ATCC (American Type Culture Collection), were suspended into Matrigel matrix, subcutaneously transplanted at $1.0 \times 10^7$ cells into the right side of female nude mice, and the mice were randomly grouped 7 days after the transplantation (Day 0). The HER2-ADC (1) was intravenously administered to the tail vein at a dose of 0.5 mg/kg on Day 0. Pertuzumab was intravenously administered to the tail vein at a dose of 30 mg/kg on Day 0 and at a dose of 15 mg/kg on Day 7 and Day 14. Single administration groups of each drug, a combined administration group, and a solvent administration group as a control group were set up.

Results of a combination of HER2-ADC (1) and pertuzumab are shown in Figure 14. Single administration of pertuzumab showed TGI of 95% on the day of determination of effect, and loss of tumor was found in three of six cases. Single administration of HER2-ADC (1) showed TGI of 74%, and loss of tumor was not found in any of six cases. On the other hand, combined administration of HER2-ADC (1) and pertuzumab exhibited a significantly superior tumor growth suppression effect than single administration of HER2-ADC (1) ($P < 0.001$) and showed TGI of 99%, and loss of tumor was found in five of six cases. In addition, none of the single and combined administration groups exhibited any particular notable finding such as weight loss.

Free Text of Sequence Listing

SEQ ID NO: 1 - Amino acid sequence of a heavy chain of the anti-HER2 antibody
SEQ ID NO: 2 - Amino acid sequence of a light chain of the anti-HER2 antibody
SEQ ID NO: 3 - Amino acid sequence of CDRH1 of an anti-HER2 antibody
SEQ ID NO: 4 - Amino acid sequence of CDRH2 of an anti-HER2 antibody
SEQ ID NO: 5 - Amino acid sequence of CDRH3 of an anti-HER2 antibody
SEQ ID NO: 6 - amino acid sequence of CDRL1 of an anti-HER2 antibody
SEQ ID NO: 7 - Amino acid sequence including the amino acid sequence of CDRL2 (SAS) of the anti-HER2 antibody
SEQ ID NO: 8 - Amino acid sequence of CDRL3 of the anti-HER2 antibody
SEQ ID NO: 9 - Amino acid sequence of a heavy chain variable region of the anti-HER2 antibody
SEQ ID NO: 10 - Amino acid sequence of a light chain variable region of the anti-HER2 antibody
SEQ ID NO: 11 - Amino acid sequence of a heavy chain of the anti-HER2 antibody
SEQ ID NO: 12 - Amino acid sequence of HER2 protein
SEQ ID NO: 13 - Nucleotide sequence of a HER2 gene (cDNA)

## Claims

**1.** A pharmaceutical composition, wherein

an anti-HER2 antibody-drug conjugate and a HER dimerization inhibitor are administered in combination, and the anti-HER2 antibody-drug conjugate is an anti-HER2 antibody-drug conjugate in which a drug-linker represented by the following formula:

[Formula 1]

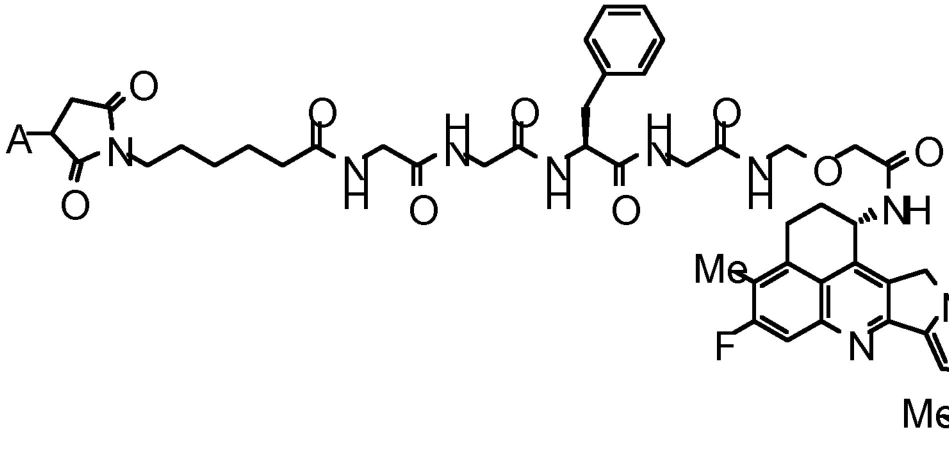

wherein A represents a connecting position to an anti-HER2 antibody, is conjugated to the anti-HER2 antibody via a thioether bond.

2. The pharmaceutical composition according to claim 1, wherein the anti-HER2 antibody is an antibody comprising a heavy chain comprising CDRH1 consisting of an amino acid sequence represented by SEQ ID NO: 3, CDRH2 consisting of an amino acid sequence represented by SEQ ID NO: 4, and CDRH3 consisting of an amino acid sequence represented by SEQ ID NO: 5, and a light chain comprising CDRL1 consisting of an amino acid sequence represented by SEQ ID NO: 6, CDRL2 consisting of an amino acid sequence represented by amino acid residues 1 to 3 of SEQ ID NO: 7, and CDRL3 consisting of an amino acid sequence represented by SEQ ID NO: 8.

3. The pharmaceutical composition according to claim 1, wherein the anti-HER2 antibody is an antibody comprising a heavy chain comprising a heavy chain variable region consisting of an amino acid sequence represented by SEQ ID NO: 9 and a light chain comprising a light chain variable region consisting of an amino acid sequence represented by SEQ ID NO: 10.

4. The pharmaceutical composition according to claim 1, wherein the anti-HER2 antibody is an antibody comprising a heavy chain consisting of an amino acid sequence represented by SEQ ID NO: 1 and a light chain consisting of an amino acid sequence represented by SEQ ID NO: 2.

5. The pharmaceutical composition according to claim 1, wherein the anti-HER2 antibody is an antibody comprising a heavy chain consisting of an amino acid sequence represented by SEQ ID NO: 11 and a light chain consisting of an amino acid sequence represented by SEQ ID NO: 2.

6. The pharmaceutical composition according to any one of claims 1 to 5, wherein the anti-HER2 antibody-drug conjugate is an anti-HER2 antibody-drug conjugate represented by the following formula:

[Formula 2]

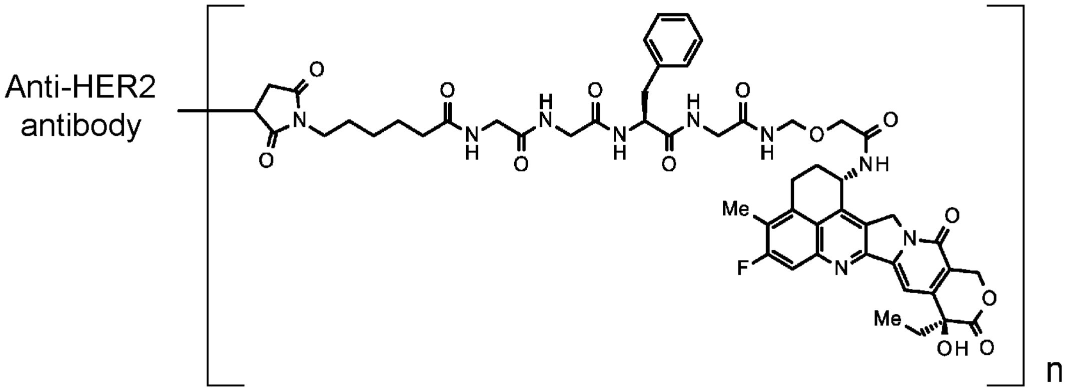

wherein a drug-linker is conjugated to the anti-HER2 antibody via a thioether bond; and n represents the average number of units of the drug-linker conjugated per antibody molecule, wherein n is in the range of from 7 to 8.

**7.** The pharmaceutical composition according to any one of claims 1 to 6, wherein the anti-HER2 antibody-drug conjugate is trastuzumab deruxtecan.

**8.** The pharmaceutical composition according to any one of claims 1 to 7, wherein the HER dimerization inhibitor is an antibody capable of binding to subdomain II of an extracellular domain of HER2 protein.

**9.** The pharmaceutical composition according to any one of claims 1 to 7, wherein the HER dimerization inhibitor is pertuzumab.

**10.** The pharmaceutical composition according to any one of claims 1 to 9, wherein the anti-HER2 antibody-drug conjugate and the HER dimerization inhibitor are separately contained as active components in different formulations, and are administered simultaneously or at different times.

**11.** The pharmaceutical composition according to any one of claims 1 to 9, wherein the anti-HER2 antibody-drug conjugate and the HER dimerization inhibitor are contained as active components in a single formulation and administered.

**12.** The pharmaceutical composition according to any one of claims 1 to 11, wherein the pharmaceutical composition is for use in treating cancer.

**13.** The pharmaceutical composition according to claim 12, wherein the cancer is at least one selected from the group consisting of breast cancer, gastric cancer, colorectal cancer, lung cancer, esophageal cancer, head-and-neck cancer, gastroesophageal junction adenocarcinoma, biliary tract cancer, Paget's disease, pancreatic cancer, ovarian cancer, uterine carcinosarcoma, bladder cancer, prostate cancer, urothelial cancer, gastrointestinal stromal tumor, uterine cervix cancer, squamous cell carcinoma, peritoneal cancer, liver cancer, hepatocellular cancer, endometrial cancer, kidney cancer, vulval cancer, thyroid cancer, penis cancer, leukemia, malignant lymphoma, plasmacytoma, myeloma, glioblastoma multiforme, sarcoma, osteosarcoma, and melanoma.

**14.** The pharmaceutical composition according to claim 12, wherein the cancer is at least one selected from the group consisting of breast cancer, gastric cancer, colorectal cancer, lung cancer, esophageal cancer, head-and-neck cancer, gastroesophageal junction adenocarcinoma, biliary tract cancer, Paget's disease, pancreatic cancer, ovarian cancer, uterine carcinosarcoma, bladder cancer, and prostate cancer.

**15.** The pharmaceutical composition according to claim 12, wherein the cancer is breast cancer.

**16.** The pharmaceutical composition according to any one of claims 12 to 15, wherein the cancer is HER2-overexpressing cancer.

**17.** The pharmaceutical composition according to claim 16, wherein the HER2-overexpressing cancer is cancer given a score of 3+ for the expression of HER2 in an immunohistochemical method.

**18.** The pharmaceutical composition according to claim 16, wherein the HER2-overexpressing cancer is cancer given a score of 2+ for the expression of HER2 in an immunohistochemical method and determined as positive for the expression of HER2 in an *in situ* hybridization method.

**19.** The pharmaceutical composition according to any one of claims 12 to 15, wherein the cancer is HER2 low-expressing cancer.

**20.** The pharmaceutical composition according to claim 19, wherein the HER2 low-expressing cancer is cancer given a score of 2+ for the expression of HER2 in an immunohistochemical method and determined as negative for the expression of HER2 in an *in situ* hybridization method.

**21.** The pharmaceutical composition according to claim 19, wherein the HER2 low-expressing cancer is cancer given a score of 1+ for the expression of HER2 in an immunohistochemical method.

**22.** The pharmaceutical composition according to claim 19, wherein the HER2 low-expressing cancer is cancer given a score of >0 and <1+ for the expression of HER2 in an immunohistochemical method.

**23.** The pharmaceutical composition according to any one of claims 12 to 22, wherein the pharmaceutical composition exerts a significantly superior antitumor effect than single administration of the anti-HER2 antibody-drug conjugate.

**24.** The pharmaceutical composition according to any one of claims 12 to 23, wherein the pharmaceutical composition is for use in treating cancer untreatable with single administration of the anti-HER2 antibody-drug conjugate.

**25.** The pharmaceutical composition according to any one of claims 12 to 24, wherein the anti-HER2 antibody-drug conjugate and the HER dimerization inhibitor exert a synergistic antitumor effect.

**26.** A method of treatment, comprising administering an anti-HER2 antibody-drug conjugate and a HER dimerization inhibitor in combination to a subject in need of the treatment, wherein the anti-HER2 antibody-drug conjugate is an anti-HER2 antibody-drug conjugate in which a drug-linker represented by the following formula:

[Formula 3]

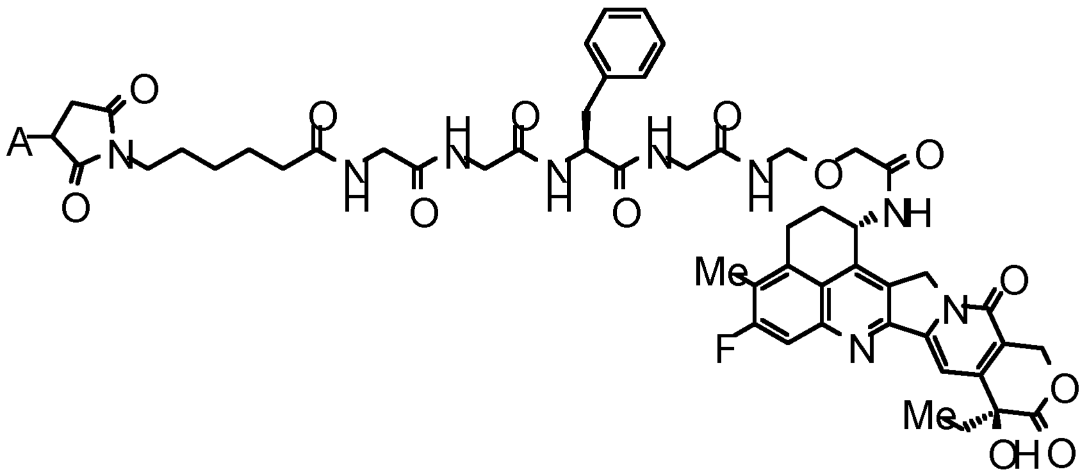

wherein A represents a connecting position to an anti-HER2 antibody,
is conjugated to the anti-HER2 antibody via a thioether bond.

27. The method of treatment according to claim 26, wherein the anti-HER2 antibody is an antibody comprising a heavy chain comprising CDRH1 consisting of an amino acid sequence represented by SEQ ID NO: 3, CDRH2 consisting of an amino acid sequence represented by SEQ ID NO: 4, and CDRH3 consisting of an amino acid sequence represented by SEQ ID NO: 5, and a light chain comprising CDRL1 consisting of an amino acid sequence represented by SEQ ID NO: 6, CDRL2 consisting of an amino acid sequence represented by amino acid residues 1 to 3 of SEQ ID NO: 7, and CDRL3 consisting of an amino acid sequence represented by SEQ ID NO: 8.

28. The method of treatment according to claim 26, wherein the anti-HER2 antibody is an antibody comprising a heavy chain comprising a heavy chain variable region consisting of an amino acid sequence represented by SEQ ID NO: 9 and a light chain comprising a light chain variable region consisting of an amino acid sequence represented by SEQ ID NO: 10.

29. The method of treatment according to claim 26, wherein the anti-HER2 antibody is an antibody comprising a heavy chain consisting of an amino acid sequence represented by SEQ ID NO: 1 and a light chain consisting of an amino acid sequence represented by SEQ ID NO: 2.

30. The method of treatment according to claim 26, wherein the anti-HER2 antibody is an antibody comprising a heavy chain consisting of an amino acid sequence represented by SEQ ID NO: 11 and a light chain consisting of an amino acid sequence represented by SEQ ID NO: 2.

31. The method of treatment according to any one of claims 26 to 30, wherein the anti-HER2 antibody-drug conjugate is an anti-HER2 antibody-drug conjugate represented by the following formula:

[Formula 4]

Anti-HER2 antibody

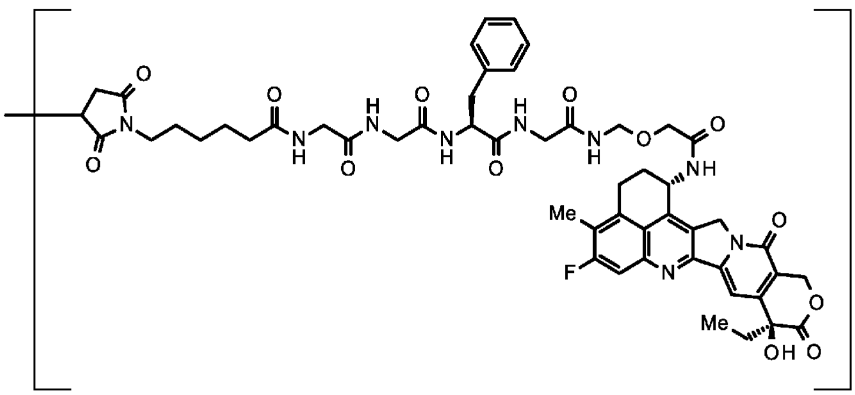

n

wherein a drug-linker is conjugated to the anti-HER2 antibody via a thioether bond; and n represents the average number of units of the drug-linker conjugated per antibody molecule, wherein n is in the range of from 7 to 8.

**32.** The method of treatment according to any one of claims 26 to 31, wherein the anti-HER2 antibody-drug conjugate is trastuzumab deruxtecan.

**33.** The method of treatment according to any one of claims 26 to 32, wherein the HER dimerization inhibitor is an antibody capable of binding to subdomain II of an extracellular domain of HER2 protein.

**34.** The method of treatment according to any one of claims 26 to 32, wherein the HER dimerization inhibitor is pertuzumab.

**35.** The method of treatment according to any one of claims 26 to 34, wherein the anti-HER2 antibody-drug conjugate and the HER dimerization inhibitor are separately contained as active components in different formulations, and are administered simultaneously or at different times.

**36.** The method of treatment according to any one of claims 26 to 34, wherein the anti-HER2 antibody-drug conjugate and the HER dimerization inhibitor are contained as active components in a single formulation and administered.

**37.** The method of treatment according to any one of claims 26 to 36, wherein the method is for use in treating cancer.

**38.** The method of treatment according to claim 37, wherein the cancer is at least one selected from the group consisting of breast cancer, gastric cancer, colorectal cancer, lung cancer, esophageal cancer, head-and-neck cancer, gastroesophageal junction adenocarcinoma, biliary tract cancer, Paget's disease, pancreatic cancer, ovarian cancer, uterine carcinosarcoma, bladder cancer, prostate cancer, urothelial cancer, gastrointestinal stromal tumor, uterine cervix cancer, squamous cell carcinoma, peritoneal cancer, liver cancer, hepatocellular cancer, endometrial cancer, kidney cancer, vulval cancer, thyroid cancer, penis cancer, leukemia, malignant lymphoma, plasmacytoma, myeloma, glioblastoma multiforme, sarcoma, osteosarcoma, and melanoma.

**39.** The method of treatment according to claim 37, wherein the cancer is at least one selected from the group consisting of breast cancer, gastric cancer, colorectal cancer, lung cancer, esophageal cancer, head-and-neck cancer, gastroesophageal junction adenocarcinoma, biliary tract cancer, Paget's disease, pancreatic cancer, ovarian cancer, uterine carcinosarcoma, bladder cancer, and prostate cancer.

**40.** The method of treatment according to claim 37, wherein the cancer is breast cancer.

**41.** The method of treatment according to any one of claims 37 to 40, wherein the cancer is HER2-overexpressing cancer.

**42.** The method of treatment according to claim 41, wherein the HER2-overexpressing cancer is cancer given a score of 3+ for the expression of HER2 in an immunohistochemical method.

**43.** The method of treatment according to claim 41, wherein the HER2-overexpressing cancer is cancer given a score of 2+ for the expression of HER2 in an immunohistochemical method and determined as positive for the expression of HER2 in an *in situ* hybridization method.

**44.** The method of treatment according to any one of claims 37 to 40, wherein the cancer is HER2 low-expressing cancer.

**45.** The method of treatment according to claim 44, wherein the HER2 low-expressing cancer is cancer given a score of 2+ for the expression of HER2 in an immunohistochemical method and determined as negative for the expression of HER2 in an *in situ* hybridization method.

**46.** The method of treatment according to claim 44, wherein the HER2 low-expressing cancer is cancer given a score of 1+ for the expression of HER2 in an immunohistochemical method.

**47.** The method of treatment according to claim 44, wherein the HER2 low-expressing cancer is cancer given a score of >0 and <1+ for the expression of HER2 in an immunohistochemical method.

**48.** The method of treatment according to any one of claims 37 to 47, which exerts a significantly superior antitumor

effect than single administration of the anti-HER2 antibody-drug conjugate.

**49.** The method of treatment according to any one of claims 37 to 48, wherein the method is for use in treating cancer untreatable with single administration of the anti-HER2 antibody-drug conjugate.

**50.** The method of treatment according to any one of claims 37 to 49, wherein the anti-HER2 antibody-drug conjugate and the HER dimerization inhibitor exert a synergistic antitumor effect.

[Figure 1]

SEQ ID No: 1 - Amino acid sequence of a heavy chain of the humanized anti-HER2 antibody

EVQLVESGGGLVQPGGSLRLSCAASGFNIKDTYIHWVRQAPGKGLEWVARI
YPTNGYTRYADSVKGRFTISADTSKNTAYLQMNSLRAEDTAVYYCSRWGG
DGFYAMDYWGQGTLVTVSSASTKGPSVFPLAPSSKSTSGGTAALGCLVKDY
FPEPVTVSWNSGALTSGVHTFPAVLQSSGLYSLSSVVTVPSSSLGTQTYICNV
NHKPSNTKVDKKVEPKSCDKTHTCPPCPAPELLGGPSVFLFPPKPKDTLMISR
TPEVTCVVVDVSHEDPEVKFNWYVDGVEVHNAKTKPREEQYNSTYRVVSV
LTVLHQDWLNGKEYKCKVSNKALPAPIEKTISKAKGQPREPQVYTLPPSREE
MTKNQVSLTCLVKGFYPSDIAVEWESNGQPENNYKTTPPVLDSDGSFFLYSK
LTVDKSRWQQGNVFSCSVMHEALHNHYTQKSLSLSPGK

[Figure 2]

SEQ ID No: 2 - Amino acid sequence of a light chain of the humanized anti-HER2 antibody

DIQMTQSPSSLSASVGDRVTITCRASQDVNTAVAWYQQKPGKAPKLLIYSAS
FLYSGVPSRFSGSRSGTDFTLTISSLQPEDFATYYCQQHYTTPPTFGQGTKVEI
KRTVAAPSVFIFPPSDEQLKSGTASVVCLLNNFYPREAKVQWKVDNALQSG
NSQESVTEQDSKDSTYSLSSTLTLSKADYEKHKVYACEVTHQGLSSPVTKSF
NRGEC

[Figure 3]

SEQ ID No: 3 - Amino acid sequence of CDRH1 of the anti-HER2 antibody

GFNIKDTY

[Figure 4]

SEQ ID No: 4 - Amino acid sequence of CDRH2 of the anti-HER2 antibody

IYPTNGYT

[Figure 5]

SEQ ID No: 5 - Amino acid sequence of CDRH3 of the anti-HER2 antibody

SRWGGDGFYAMDY

[Figure 6]

SEQ ID No: 6 - Amino acid sequence of CDRL1 of the anti-HER2 antibody

QDVNTA

[Figure 7]

SEQ ID No: 7 - Amino acid sequence including the amino acid sequence of CDRL2 (SAS) of the anti-HER2 antibody

SASFLYS

[Figure 8]

SEQ ID No: 8 - Amino acid sequence of CDRL3 of the anti-HER2 antibody

QQHYTTPPT

[Figure 9]

SEQ ID No: 9 - Amino acid sequence of a heavy chain variable region of the anti-HER2 antibody

EVQLVESGGGLVQPGGSLRLSCAASGFNIKDTYIHWVRQAPGKGLEWVARI
YPTNGYTRYADSVKGRFTISADTSKNTAYLQMNSLRAEDTAVYYCSRWGG
DGFYAMDYWGQGTLVTVSS

[Figure 10]

SEQ ID No: 10 - Amino acid sequence of a light chain variable region of the anti-HER2 antibody

DIQMTQSPSSLSASVGDRVTITCRASQDVNTAVAWYQQKPGKAPKLLIYSAS
FLYSGVPSRFSGSRSGTDFTLTISSLQPEDFATYYCQQHYTTPPTFGQGTKVEI
K

[Figure 11]

SEQ ID No: 11 - Amino acid sequence of a heavy chain of the anti-HER2 antibody

EVQLVESGGGLVQPGGSLRLSCAASGFNIKDTYIHWVRQAPGKGLEWVARI
YPTNGYTRYADSVKGRFTISADTSKNTAYLQMNSLRAEDTAVYYCSRWGG
DGFYAMDYWGQGTLVTVSSASTKGPSVFPLAPSSKSTSGGTAALGCLVKDY
FPEPVTVSWNSGALTSGVHTFPAVLQSSGLYSLSSVVTVPSSSLGTQTYICNV
NHKPSNTKVDKKVEPKSCDKTHTCPPCPAPELLGGPSVFLFPPKPKDTLMISR
TPEVTCVVVDVSHEDPEVKFNWYVDGVEVHNAKTKPREEQYNSTYRVVSV
LTVLHQDWLNGKEYKCKVSNKALPAPIEKTISKAKGQPREPQVYTLPPSREE
MTKNQVSLTCLVKGFYPSDIAVEWESNGQPENNYKTTPPVLDSDGSFFLYSK
LTVDKSRWQQGNVFSCSVMHEALHNHYTQKSLSLSPG

[Figure 12]

SEQ ID No: 12 - Amino acid sequence of HER2 protein

MELAALCRWGLLLALLPPGAASTQVCTGTDMKLRLPASPETHLDMLRHLY
QGCQVVQGNLELTYLPTNASLSFLQDIQEVQGYVLIAHNQVRQVPLQRLRIV
RGTQLFEDNYALAVLDNGDPLNNTTPVTGASPGGLRELQLRSLTEILKGGVL
IQRNPQLCYQDTILWKDIFHKNNQLALTLIDTNRSRACHPCSPMCKGSRCWG
ESSEDCQSLTRTVCAGGCARCKGPLPTDCCHEQCAAGCTGPKHSDCLACLH
FNHSGICELHCPALVTYNTDTFESMPNPEGRYTFGASCVTACPYNYLSTDVG
SCTLVCPLHNQEVTAEDGTQRCEKCSKPCARVCYGLGMEHLREVRAVTSAN
IQEFAGCKKIFGSLAFLPESFDGDPASNTAPLQPEQLQVFETLEEITGYLYISA

WPDSLPDLSVFQNLQVIRGRILHNGAYSLTLQGLGISWLGLRSLRELGSGLAL
IHHNTHLCFVHTVPWDQLFRNPHQALLHTANRPEDECVGEGLACHQLCARG
HCWGPGPTQCVNCSQFLRGQECVEECRVLQGLPREYVNARHCLPCHPECQP
QNGSVTCFGPEADQCVACAHYKDPPFCVARCPSGVKPDLSYMPIWKFPDEE
GACQPCPINCTHSCVDLDDKGCPAEQRASPLTSIISAVVGILLVVVLGVVFGIL
IKRRQQKIRKYTMRRLLQETELVEPLTPSGAMPNQAQMRILKETELRKVKVL
GSGAFGTVYKGIWIPDGENVKIPVAIKVLRENTSPKANKEILDEAYVMAGVG
SPYVSRLLGICLTSTVQLVTQLMPYGCLLDHVRENRGRLGSQDLLNWCMQI
AKGMSYLEDVRLVHRDLAARNVLVKSPNHVKITDFGLARLLDIDETEYHAD
GGKVPIKWMALESILRRRFTHQSDVWSYGVTVWELMTFGAKPYDGIPAREI
PDLLEKGERLPQPPICTIDVYMIMVKCWMIDSECRPRFRELVSEFSRMARDPQ
RFVVIQNEDLGPASPLDSTFYRSLLEDDDMGDLVDAEEYLVPQQGFFCPDPA
PGAGGMVHHRHRSSSTRSGGGDLTLGLEPSEEEAPRSPLAPSEGAGSDVFDG
DLGMGAAKGLQSLPTHDPSPLQRYSEDPTVPLPSETDGYVAPLTCSPQPEYV
NQPDVRPQPPSPREGPLPAARPAGATLERPKTLSPGKNGVVKDVFAFGGAVE
NPEYLTPQGGAAPQPHPPPAFSPAFDNLYYWDQDPPERGAPPSTFKGTPTAE
NPEYLGLDVPV

[Figure 13]

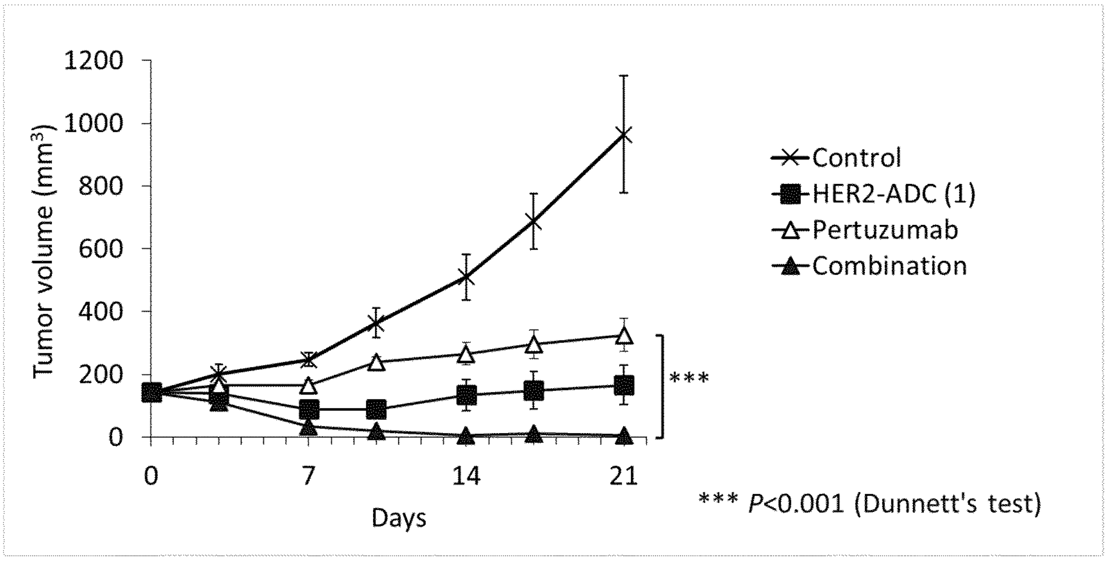


[Figure 14]

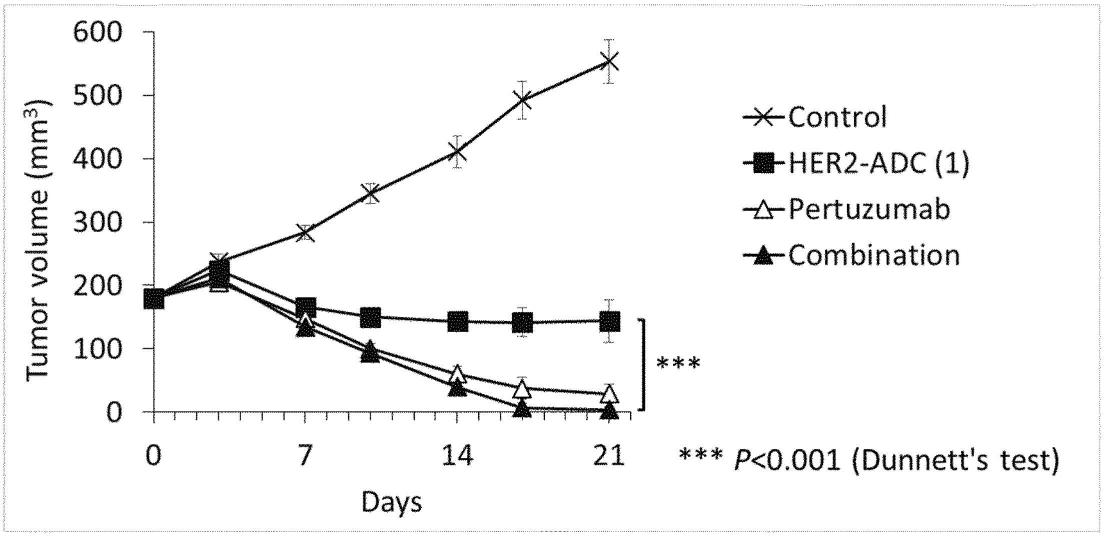

**INTERNATIONAL SEARCH REPORT**

International application No.
**PCT/JP2021/026932**

**A. CLASSIFICATION OF SUBJECT MATTER**

***A61K 47/68***(2017.01)i; ***A61K 31/4745***(2006.01)i; ***A61K 39/395***(2006.01)i; ***A61P 35/00***(2006.01)i; ***A61P 35/02***(2006.01)i; ***A61P 43/00***(2006.01)i; ***C07K 16/30***(2006.01)i; ***C12N 15/13***(2006.01)i
FI: A61K47/68; A61K31/4745 ZNA; A61K39/395 L; A61K39/395 T; A61P35/00; A61P35/02; A61P43/00 121; C07K16/30; C12N15/13

According to International Patent Classification (IPC) or to both national classification and IPC

**B. FIELDS SEARCHED**

Minimum documentation searched (classification system followed by classification symbols)

A61K47/68; A61K31/4745; A61K39/395; A61P35/00; A61P35/02; A61P43/00; C07K16/30; C12N15/13

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| Y | WO 2015/115091 A1 (DAIICHI SANKYO CO., LTD.) 06 August 2015 (2015-08-06) claims, example 50, evaluation example 7, etc. | 1-50 |
| Y | WO 2020/022363 A1 (DAIICHI SANKYO CO., LTD.) 30 January 2020 (2020-01-30) claims, paragraph [0261], etc. | 1-50 |
| Y | RICHARD, Sandrine et al. Pertuzumab and trastuzumab: the rationale way to synergy. Annals of the Brazilian Academy of Sciences, 2016, 88(1 Suppl.), pp. 565-577, http://dx.doi.org/10.1590/0001-3765201620150178 abstract, etc. | 1-50 |
| Y | NAMI, Babak et al. The Effects of Pertuzumab and Its Combination with Trastuzumab on HER2 Homodimerization and Phosphorylation, Cancers, 2019, 11, 375, doi: 10.3390/cancers11030375 abstract, etc. | 1-50 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **15 September 2021** | **28 September 2021** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2021/026932**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**
**Information on patent family members**

International application No.
**PCT/JP2021/026932**

| Patent document cited in search report | Publication date (day/month/year) | Patent family member(s) | Publication date (day/month/year) |
|---|---|---|---|
| WO 2015/115091 A1 | 06 August 2015 | US 2016/0333112 A1<br>claims, example 50, evaluation example 7<br>US 2019/0077880 A1<br>EP 3101032 A1<br>EP 3466976 A1<br>CN 105829346 A<br>KR 10-2016-0113099 A | |
| WO 2020/022363 A1 | 30 January 2020 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5821337 A
- WO 0100245 A
- WO 2015115091 A
- WO 2015155976 A
- WO 2018066626 A
- WO 2019230645 A
- WO 9007861 A
- WO 9954342 A
- WO 0061739 A
- WO 0231140 A


### Non-patent literature cited in the description


- **COUSSENS L et al.** *Science,* 1985, vol. 230 (4730), 1132-1139
- **GRAUS-PORTA G et al.** *EMBO J.,* 1997, vol. 16, 1647-1655
- **KARNAGARAN D et al.** *EMBO J.,* 1996, vol. 15, 254-264
- **SLIWKOWSKI MX et al.** *J Biom Chem.,* 1994, vol. 269, 14661-14665
- **DI FORE PP et al.** *Science,* 1987, vol. 237, 178-182
- **HUDZIAK RM et al.** *Proc Natl Acad Sci USA.,* 1987, vol. 84, 7159-7163
- **HARDWICK R et al.** *Eur. J Surg Oncol.,* 1997, vol. 23, 30-35
- **KORKAYA H et al.** *Oncogene,* 2008, vol. 27 (47), 6120-6130
- **YANO T et al.** *Oncol Rep.,* 2006, vol. 15 (1), 65-71
- **SLAMON DJ et al.** *Science,* 1987, vol. 235, 177-182
- **GRAVALOS C et al.** *Ann Oncol,* 2008, vol. 19, 1523-1529
- **FUKUSHIGE S et al.** *Mol Cell Biol,* 1986, vol. 6, 955-958
- **SLAMON DJ et al.** *Science,* 1989, vol. 244, 707-712
- **KAPTAIN S et al.** *Diagn Mol Pathol,* 2001, vol. 10, 139-152
- **RICHARD S et al.** *An Acad Bras Cienc.,* 2016, vol. 88 (1), 565-77
- **NAMI B et al.** *Cancers (Basel).,* 16 March 2019, vol. 11 (3), 375
- **DUCRY, L. et al.** *Bioconjugate Chem.,* 2010, vol. 21, 5-13
- **ALLEY, S. C. et al.** *Current Opinion in Chemical Biology,* 2010, vol. 14, 529-537
- **DAMLE N. K.** *Expert Opin. Biol. Ther.,* 2004, vol. 4, 1445-1452
- **SENTER P. D. et al.** *Nature Biotechnology,* 2012, vol. 30, 631-637
- **HOWARD A. et al.** *J Clin Oncol,* vol. 29, 398-405
- **OGITANI Y. et al.** *Clinical Cancer Research,* 2016, vol. 22 (20), 5097-5108
- **OGITANI Y. et al.** *Cancer Science,* 2016, vol. 107, 1039-1046
- **DOI T et al.** *Lancet Oncol.,* 2017, vol. 18, 1512-22
- **TAKEGAWA N et al.** *Int. J. Cancer,* 2017, vol. 141, 1682-1689
- *Oncol Rep.,* September 2013, vol. 30 (3), 1087-93
- *Clin Cancer Res.,* 15 January 2014, vol. 20 (2), 456-68
- *Oncotarget.,* 07 August 2018, vol. 9 (61), 31915-31919
- **OGITANI Y. et al.** *Clinical Cancer Research,* 29 March 2016, vol. 22 (20), 5097-5108
- **KOHLER ; MILSTEIN.** *Nature,* 1975, vol. 256, 495-497
- Monoclonal Antibodies. Plenum Press, 1980, 365-367
- *Proc. Natl. Acad. Sci. USA,* 1984, vol. 81, 6851-6855
- *Nature,* 1986, vol. 321, 522-525
- **TOMIZUKA, K. et al.** *Nature Genetics,* 1997, vol. 16, 133-143
- **KUROIWA, Y.** *Nucl. Acids Res.,* 1998, vol. 26, 3447-3448
- **YOSHIDA, H.** Animal Cell Technology: Basic and Applied Aspects. Kluwer Academic Publishers, 1999, vol. 10, 69-73
- **TOMIZUKA, K.** *Proc. Natl. Acad. Sci. USA,* 2000, vol. 97, 722-727
- **WORMSTONE, I. M.** *Investigative Ophthalmology & Visual Science,* 2002, vol. 43 (7), 2301-2308

- **CARMEN, S.** *Briefings in Functional Genomics and Proteomics,* 2002, vol. 1 (2), 189-203
- **SIRIWARDENA, D.** *Ophthalmology,* 2002, vol. 109 (3), 427-431
- *Journal of Chromatography A,* 1995, vol. 705, 129-134
- *Analytical Biochemistry,* 2007, vol. 360, 75-83
- **HERMANSON, G. T.** Bioconjugate Techniques. Academic Press, 1996, 456-493
- *British Journal of Cancer,* 1999, vol. 79 (5/6), 707-717
- *Breast Cancer Res.,* 12 August 2011, vol. 13 (4), 215
- *J Mol Diagn.,* March 2017, vol. 19 (2), 244-254